# EUROPEAN PATENT SPECIFICATION

(11) **EP 4 272 751 B1**
(45) Date of publication and mention of the grant of the patent: **24.09.2025**
(21) Application number: 23192007.5
(22) Date of filing: 26.08.2020
(51) Int. Cl.: C07K 7/08, C07K 14/47, A61K 38/00

(54) **COMPSTATIN ANALOGUES AND THEIR MEDICAL USES**
COMPSTATIN-ANALOGA UND DEREN MEDIZINISCHE VERWENDUNGEN
ANALOGUES DE COMPSTATINE ET LEURS UTILISATIONS MÉDICALES

(30) Priority: 27.08.2019 EP 19193925; 29.04.2020 EP 20172189
(43) Date of publication of application: 08.11.2023
(62) Divisional of application: 20760472.9
(73) Proprietor: ZP SPV 3 K/S, 2860 Søborg (DK)
(72) Inventor: SHELTON, Anne, Pernile, Tofteng, 2860 Søborg (DK); MUNCH, Henrik, Fischer, 2860 Søborg (DK)
(74) Representative: Mewburn Ellis LLP

(56) References cited:
- WO-A1-2010/039690
- WO-A1-2014/100407
- WO-A1-2019/166411
- WO-A1-99/13899
- WO-A2-2012/040259
- WO-A2-2013/036778
- US-A1- 2018 057 538
- QU HONGCHANG ET AL: "New analogs of the clinical complement inhibitor compstatin with subnanomolar affinity and enhanced pharmacokinetic properties", IMMUNOBIOLOGY, URBAN UND FISCHER VERLAG, DE, vol. 218, no. 4, 17 June 2012 (2012-06-17), pages 496 - 505, XP028991254, ISSN: 0171-2985, DOI: 10.1016/J.IMBIO.2012.06.003

## Description

### Field of the Invention

The present invention relates to inhibiting activation of the complement cascade in the body, and more particularly to compstatin analogues that are capable of binding to C3 protein and inhibiting complement activation. The present invention also relates to the medical uses of the compstatin analogues, in particular for the treatment of conditions characterized by unwanted activation of the complement cascade, such as autoimmune and inflammatory diseases.

### Background of the Invention

The human complement system is a powerful player in the defense against pathogenic organisms and the mediation of immune responses. Complement can be activated through three different pathways: the classical, lectin and alternative pathways. The major activation event that is shared by all three pathways is the proteolytic cleavage of the central protein of the complement system, C3, into its activation products C3a and C3b by C3 convertases. Generation of these fragments leads to the opsonization of pathogenic cells by C3b and iC3b, a process that renders them susceptible to phagocytosis or clearance, and to the activation of immune cells through an interaction with complement receptors (Markiewski & Lambris, 2007, Am. J. Pathol., 171: 715-727). Deposition of C3b on target cells also induces the formation of new convertase complexes and thereby initiates a self-amplification loop. An ensemble of plasma and cell surface-bound proteins carefully regulates complement activation to prevent host cells from self-attack by the complement cascade. However, excessive activation or inappropriate regulation of complement can lead to a number of pathologic conditions, ranging from autoimmune to inflammatory diseases (Holers, 2003, Clin. Immunol., 107: 140-51; Markiewski & Lambris, 2007, supra; Ricklin & Lambris, 2007, Nat. Biotechnol., 25: 1265-75; Sahu et al., 2000, J. Immunol., 165: 2491-9). The development of therapeutic complement inhibitors is therefore highly desirable. In this context, C3 and C3b have emerged as promising targets because their central role in the cascade allows for the simultaneous inhibition of the initiation, amplification, and downstream activation of complement (Ricklin & Lambris, 2007, supra).

Compstatin was first identified as a 27 amino acid peptide and was the first non-host-derived complement inhibitor that was shown to be capable of blocking all three activation pathways (Sahu et al., 1996, J. Immunol., 157: 884-91; U.S. Patent No: 6,319,897). It has been shown that it is possible to truncate compstatin without loss of activity to a 13 amino acid peptide. However, attempts to further truncate this peptide led to loss of activity. The sequence of the 13 amino acid truncated (or "core") compstatin peptide is Ile¹-Cys²-Val³-Val⁴-Gln⁵-Asp⁶-Trp⁷-Gly⁸-His⁹-His¹⁰-Arg¹¹-Cys¹²-Thr¹³-NH₂, where Cys² and Cys¹² are disulfide bonded. This cyclic tridecapeptide binds to C3 (and fragments of C3), thereby inhibiting the activation of the downstream complement cascade and preventing the cleavage of native C3 by the C3 convertases. Its inhibitory efficacy was confirmed by a series of studies using experimental models that pointed to its potential as a therapeutic agent (Fiane et al, 1999a, Xenotransplantation, 6: 52-65; Fiane et al, 1999b, Transplant Proc., 31:934- 935; Nilsson et al., 1998, Blood, 92: 1661-1667; Ricklin & Lambris, 2008, Adv. Exp. Med..Biol., 632: 273-292; Schmidt et al., 2003, J. Biomed. Mater. Res., A66: 491-499; Soulika et al., 2000, Clin. Immunol., 96: 212-221).

Progressive optimization of the 13 amino acid compstatin peptide has led to analogues with improved biological activity (Ricklin & Lambris, 2008, supra; WO 2004/026328; WO 2007/062249, WO 2013/036778, WO 2014/100407).

Earlier structure-activity studies have identified the cyclic nature of the compstatin peptide and the presence of both a β-turn and hydrophobic cluster as key features of the molecule (Morikis et al., 1998, Protein Sci., 7: 619-627; WO 99/13899; Morikis et al., 2002, J. Biol. Chem., 277:14942-14953; Ricklin & Lambris, 2008, supra). Hydrophobic residues at positions 4 and 7 were found to be of particular importance, and their modification with unnatural amino acids generated an analogue with 264-fold improved activity over the original compstatin peptide (Katragadda et al., 2006, J. Med. Chem., 49: 4616-4622; WO 2007/062249). Further attempts to optimize compstatin for use in the treatment of eye disorders are described in WO 2007/044668.

While previous optimization steps have been based on combinatorial screening studies, solution structures, and computational models (Chiu et al., 2008, Chem. Biol. Drug Des., 72: 249-256; Mulakala et al., 2007, Bioorg. Med. Chem., 15: 1638-1644; Ricklin & Lambris, 2008, supra), the publication of a co-crystal structure of compstatin complexed with the complement fragment C3c (Janssen et al., 2007, J. Biol. Chem., 282: 29241-29247; WO 2008/153963) provided a basis for initiating rational optimization. The crystal structure revealed a shallow binding site at the interface of macroglobulin (MG) domains 4 and 5 of C3c and showed that 9 of the 13 amino acids were directly involved in the binding, either through hydrogen bonds or hydrophobic interactions. As compared to the structure of the compstatin peptide in solution (Morikis et al., 1998, supra), the bound form of compstatin experienced a conformational change, with a shift in the location of the β-turn from residues 5-8 to 8-11 (Janssen et al., 2007, supra; WO 2008/153963).

In view of its therapeutic potential in AMD, C3G, PNH and other diseases, it remains a problem in the art to further optimize compstatin analogues, for example to achieve an even greater activity and/or to modulate pharmacokinetic properties, such as increased half-life in vivo and/or physicochemical properties such as increased solubility or stability .

### Summary of the Invention

Broadly, the present invention is based on work to develop a new family of compstatin analogues having improved binding and complement-inhibiting activity as compared to the 13 amino acid compstatin peptide (ICVVQDWGHHRCT (cyclic C2-C12)). In some cases, these compstatin analogues additionally possess useful physicochemical properties, such as increased solubility and pharmacokinetic properties. In particular, the present inventors found that introducing an isoleucine residue at position 3 in place of the wild type valine residue led to compstatin peptides with improved binding and complement-inhibiting activity. The present inventors further discovered that the introduction of isoleucine at position 3 enables the introduction of other modifications, for example modifications that are capable of increasing solubility, such as the introduction of glutamic acid at position 6, particular charged or polar amino acids at position 9, and/or the introduction of N-and/or C-terminal sequences. Example of such additional modifications include the replacement of Ile at position 1 with Tyr, Phe or Sar, replacement of Val at position 4 with Trp, a Trp analogue (as described herein); replacement of Asp in position 6 with Glu; replacement of His at position 9 with Ala, Glu, Asp, Lys, Ser or Arg; replacement of Arg at position 11 with Ser; replacement of Thr at position 13 with Ser, Glu, Sar or Ile. Preferred compstatin peptides including one or more of these modifications have improved solubility, for example as compared to the 13 amino acid compstatin peptide (ICVVQDWGHHRCT (cyclic C2-C12)). Further examples of these compstatin peptides combine modification at position 9 with extensions to the N-terminal and/or C-terminus of the peptide. Addition of acylation also has advantageous effects on pharmacokinetics.

Further, in the compounds of invention, the residues corresponding to cysteine 2 and cysteine 12 of compstatin have side chains which are linked via a thioether bond, instead of the disulfide bond found in compstatin. Amongst other advantages, it is believed that this may provide improvements in stability (e.g. physical or chemical stability) as compared to equivalent molecules containing disulfide bonds at the corresponding positions.

Accordingly, the present invention provides a compstatin analogue having the sequence:
Ac-SEFA(1)I[1-Me-Trp]QDWGEHRhC(1)TEGE-[Peg3]ES-[K*]-NH₂
wherein the side chains of residues designated A(1) and hC(1) form a cystathionine bridge, and
wherein K* is a lysine residue covalently linked to a group Z¹- or Z¹-Z²-; wherein:
   Z¹ is COOH-C₁₂₋₂₂alkylene-(CO)-; and
   Z² is selected from:
      [γGlu];
      [γGlu][Peg3][Peg3];
      [(Piperazine-1-yl)-acetyl][Peg3][Peg3];
      [yGlu]G[yGlu];
      [γGlu]K[γGlu];
      [γGlu]KG[γGlu]; or
      [yGlu]G[Peg3][yGlu][Peg3]
      where Peg3 represents 8-amino-3,6-dioxaoctanoic acid;
      or a pharmaceutically acceptable salt and/or solvate thereof.

In some embodiments, Z¹ is HOOC-(CH₂)₁₂₋₂₂-(CO)-.

In some embodiments, Z¹ is selected from:
HOOC-(CH₂)₁₂-(CO)-;
HOOC-(CH₂)₁₄-(CO)-;
HOOC-(CH₂)₁₆-(CO)-;
HOOC-(CH₂)₁₈-(CO)-; or
HOOC-(CH₂)₂₀-(CO)-; and
Z² is selected from:
   [γGlu];
   [γGlu][Peg3][Peg3]-;
   [(Piperazine-1-yl)-acetyl][Peg3][Peg3];
   [yGlu]G[yGlu];
   [γGlu]K[γGlu];
   [γGlu]KG[γGlu]; or
   [yGlu]G[Peg3][yGlu][Peg3].

For example, Z¹- or Z¹-Z²- may be selected from:
15-carboxy-pentadecanoyl;
15-carboxy-pentadecanoyl[γGlu],
15-carboxy-pentadecanoyl[γGlu][Peg3][Peg3];
19-carboxy-nonadecanoyl[γGlu][Peg3][Peg3];
15-carboxy-pentadecanoyl[(Piperazine-1-yl)-acetyl][Peg3][Peg3];
17-carboxy-heptadecanoyl[γGlu]G[γGlu];
17-carboxy-heptadecanoyl[γGlu]K[γGlu];
17-carboxy-heptadecanoyl[γGlu]KG[γGlu];
17-carboxy-heptadecanoyl[γGlu]G[Peg3][γGlu][Peg3];
15-carboxy-pentadecanoyl[yGlu]G[yGlu];
17-carboxy-heptadecanoyl;
17-carboxy-heptadecanoyl[γGlu]
19-carboxy-nonadecanoyl[γGlu]G[γGlu];and
17-carboxy-heptadecanoyl[γGlu][Peg3][Peg3].

The compstatin analogue may be:

Ac-SEFA(1)I[1-Me-Trp]QDWGEHRhC(1)TEGE[Peg3]ES-K([17-carboxy- heptadecanoyl][γGlu])-NH₂

wherein the side chains of residues designated A(1) and hC(1) form a cystathionine bridge, or a pharmaceutically acceptable salt and/or solvate thereof.

In a further aspect, the present invention provides a composition comprising a compstatin analogue of the present invention, or a pharmaceutically acceptable salt or solvate thereof, in admixture with a carrier. In some instances, the composition is a pharmaceutical composition and the carrier is a pharmaceutically acceptable carrier.

In a further aspect, the present invention provides a pharmaceutical composition comprising a compstatin analogue of the present invention, or a pharmaceutically acceptable salt or solvate thereof, in admixture with a pharmaceutically acceptable carrier, excipient or vehicle.

In a further aspect, the present invention provides a compstatin analogue of the present invention for use in therapy.

In a further aspect, the present invention provides a compstatin analogue of the present invention for use in for use in a method of prophylaxis or treatment of age-related macular degeneration, Stargardt disease, periodontitis, diabetic retinopathy, glaucoma, uveitis, rheumatoid arthritis, spinal cord injury, stroke, multiple sclerosis, Parkinson's disease, Alzheimer's disease, cancer, and respiratory disorders such as asthma, chronic obstructive pulmonary disease (COPD), allergic inflammation, emphysema, bronchitis, bronchiecstasis, cystic fibrosis, tuberculosis, pneumonia, respiratory distress syndrome (RDS - neonatal and adult), rhinitis and sinusitis; bacterial infections such as sepsis, ischemia-reperfusion injury in various tissues, myocardial infarction, anaphylaxis, paroxysmal nocturnal hemoglobinuria, autoimmune hemolytic anemias, psoriasis, hidradentitis suppurativa, myasthenia gravis, systemic lupus erythematosus, CHAPLE syndrome, C3 glomeropathy, IgA nephropathy, atypical hemolytic uremic syndrome, Crohn's disease, ulcerative colitis or antiphospholipid syndrome. It will be understood that the therapeutic effect is mediated via an effect on complement activation.

Embodiments of the present invention will now be described by way of example and not limitation.

### Description of the Figures

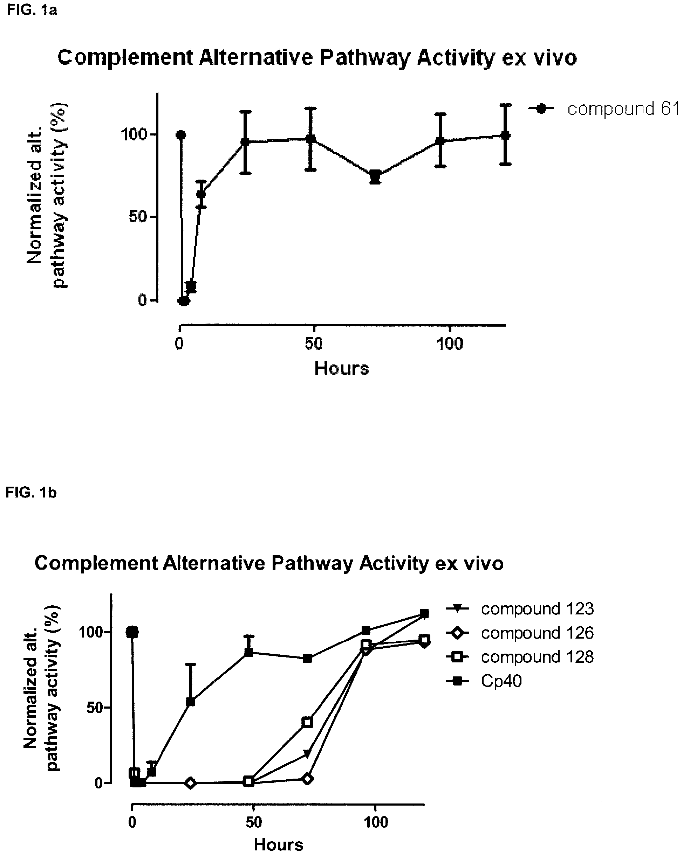

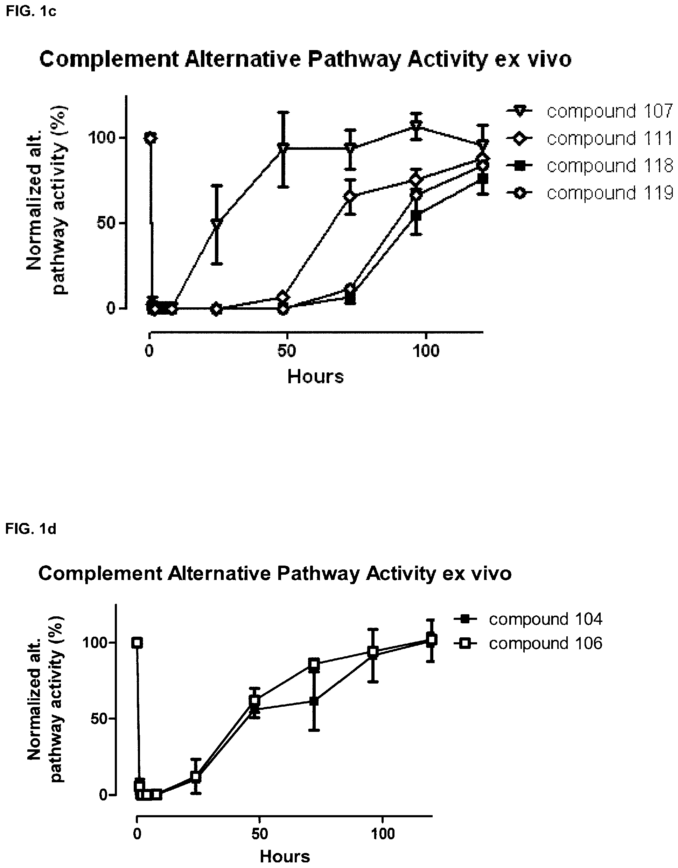

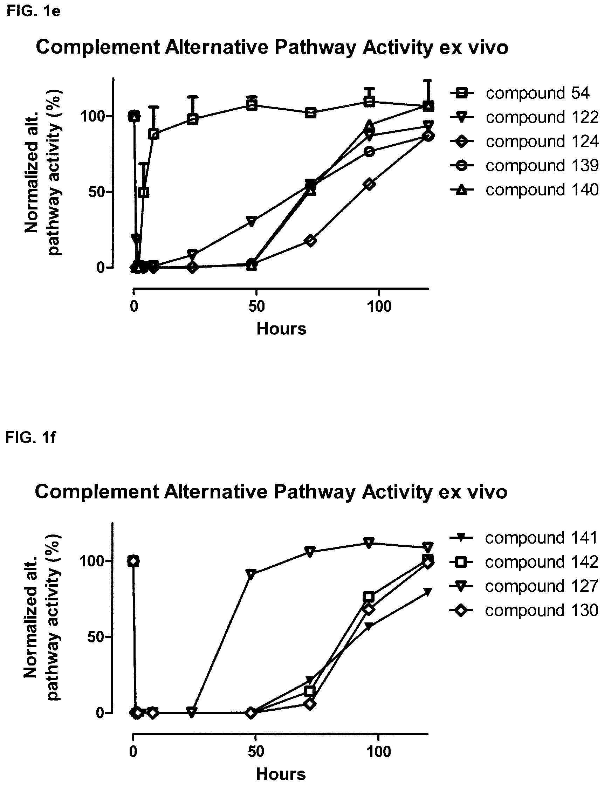

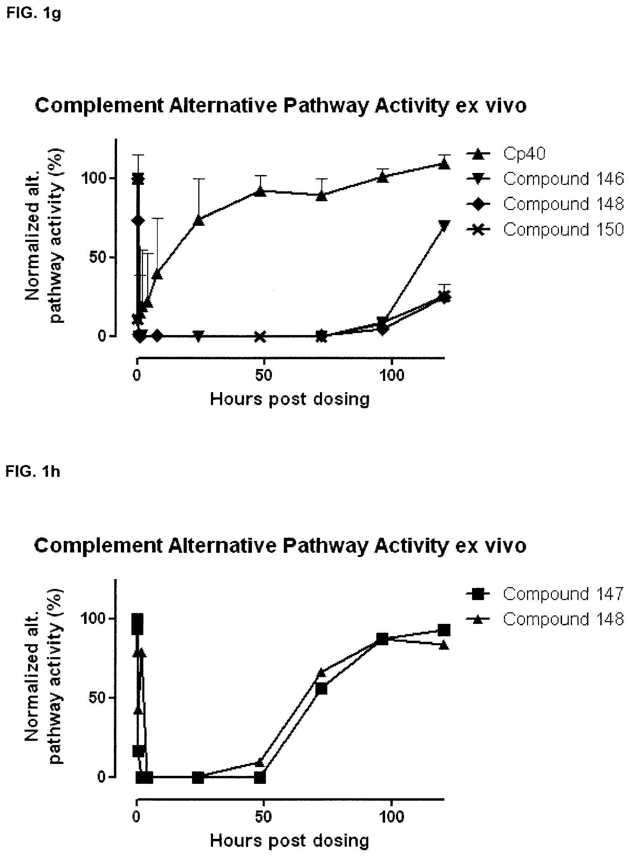

**Figure 1** **(a-g):** Normalized "ex vivo" activity of the alternative complement pathway over time after administration of a test compound at time 0 to non-human primates. Compounds were given subcutaneously at a dose of 1840 nmol/kg. Complement activity (alternative pathway) was measured using the Wieslab kit. Activity was normalized using the predose (0) sample (set to 100%) and the negative control included in the kit. Normalized activity or average normalized activity for animals and standard deviation is shown. (a) compound 61 (2 animals); (b) compound 123, compound 126 & comp 128, all with one animal per compound and Cp40 (2 animals); (c) compound 107, compound 111, compound 118 & compound 119 all with 2 animals per compound; (d) compound 104 & compound 106 with 2 animals per compound; (e) compound 54 (2 animals), and compound 122, compound 124, compound 139, and compound 140 all with 1 animal per compound; (f) compound 141, compound 142, compound 127 and compound 130, all with one animal per compound; (g) compounds 146, 148 and 150 (all one animal each), and Cp40 (four animals); (h) compounds 147 and 148 (one animal each).
**Figure 2****: Timecourses showing degradation over time of Compounds 126 and 156 at 50mg/ml in buffers F1, F2, F3:** Compound 126y: F1 (■), F2 (▲) and F3 (•) Compound 156: F1 (+), F2 (x) and F3 (◆).
**Figure 3****: Outline of the synthetic route for compound 146 (Scheme 1b)**

### Detailed Description

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

Various publications, including patents, published applications, technical articles and scholarly articles are cited throughout the specification.

Unless otherwise defined herein, scientific and technical terms used in this application shall have the meanings that are commonly understood by those of ordinary skill in the art. Generally, nomenclature used in connection with, and techniques of, chemistry, molecular biology, cell and cancer biology, immunology, microbiology, pharmacology, and protein and nucleic acid chemistry, described herein, are those well known and commonly used in the art.

Each embodiment of the invention described herein may be taken alone or in combination with one or more other embodiments of the invention.

Unless specified otherwise, the following definitions are provided for specific terms which are used in the present written description.

### Definitions

Throughout this specification, the word "comprise", and grammatical variants thereof, such as "comprises" or "comprising", will be understood to imply the inclusion of a stated integer or component, or group of integers or components, but not the exclusion of any other integer or component, or group of integers or components.

The singular forms "a," "an," and "the" include the plurals unless the context clearly dictates otherwise.

The term "including" is used to mean "including but not limited to". "Including" and "including but not limited to" may be used interchangeably.

The terms "patient", "subject" and "individual" may be used interchangeably. A subject may be a mammal, including a human or a non-human mammal, such as a non-human primate (e.g. ape, Old World monkey or New World monkey), livestock animal (e.g. bovine or porcine), companion animal (e.g. canine or feline) or laboratory animal such as a rodent (e.g. mouse or rat).

Throughout the present description and claims the conventional three-letter and one-letter codes for naturally occurring amino acids are used, i.e. A (Ala), G (Gly), L (Leu), I (lle), V (Val), F (Phe), W (Trp), S (Ser), T (Thr), Y (Tyr), N (Asn), Q (Gln), D (Asp), E (Glu), K (Lys), R (Arg), H (His), M (Met), C (Cys) and P (Pro); as well as generally accepted three-letter codes for other α-amino acids, such as norleucine (Nle), sarcosine (Sar), homocysteine (hCys; hC), α-aminoisobutyric acid (Aib), 2,3-diaminopropanoic acid (Dap), 2,4-diaminobutanoic acid (Dab) and 2,5-diaminopentanoic acid (ornithine; Orn), 1-methyl-tryptophan(1-Me-Trp, 1Me-Trp or 1MeTrp), 1-formyl-tryptophan (1-For-Trp or 1For-Trp or 1ForTrp), 1-naphthalene (1-Nal or 1Nal), 2-naphthalene (2-Nal or 2Nal), 5-methyl-tryptophan (5-Me-Trp or 5Me-Trp or 5MeTrp), p-Benzoyl-phenylalanine (Bpa) 2-indanylglycine (2Igl or 2-Igl). Other α-amino acids may be shown in square brackets "[ ]" (e.g. "[Nle]") when used in a general formula or sequence in the present specification, especially when the rest of the formula or sequence is shown using the single letter code. The 20 "naturally occurring" amino acids listed above are those which are encoded by the standard genetic code, and may also be referred to as "proteinogenic" amino acids.

Gamma-Glu and beta-Asp, also referred to as yGlu (γ-Glu) and βAsp (β-Asp) (or isoGlu and isoAsp), refers to glutamate or aspartate participating in peptide bonds via the γ- or β-carboxylic acid respectively (normally regarded as the side chain carboxyl groups), rather than the conventional configuration. Similarly, εLys or isoLys refers to lysine participating in a peptide bond via the epsilon amino group (normally regarded as the side chain amino group) rather than the alpha amino group.

Beta-Ala, also referred to as β-Ala or βAla, refers to 3-aminopropanoic acid.

Peg3 refers to a residue of 8-amino-3,6-dioxaoctanoic acid (also known as {2-[2-aminoethoxy]ethoxy}acetic acid) and Peg4 refers to a residue of 11-amino-3,6,9-trioxaundecanoic acid. The Peg3 residue may also be denoted [8-Amino-3,6-dioxaoctanoyl].

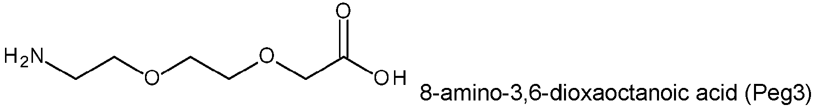

Unless otherwise specified, amino acid residues in peptides of the invention are of the L-configuration. However, in some instances, D-configuration amino acids may be incorporated. In the present context, an amino acid code written with a small letter represents the D-configuration of said amino acid, e.g. "k" represents the D-configuration of lysine (K), or a D-configuration amino acid may be written as (d)X or {d}X, where X is the amino acid, e.g. (d)Y or {d}Y represents the D-configuration of tyrosine (Y).

In compounds described herein, the side chains of the residues at positions corresponding to the cysteine residues at positions 2 and 12 of compstatin are linked by a thioether bond. For convenience, these positions may be referred to as positions X2 and X12.

Thus the side chains of the residues at X2 and X12 may together represent a cystathionine bridge. A cystathionine bridge may have two orientations: 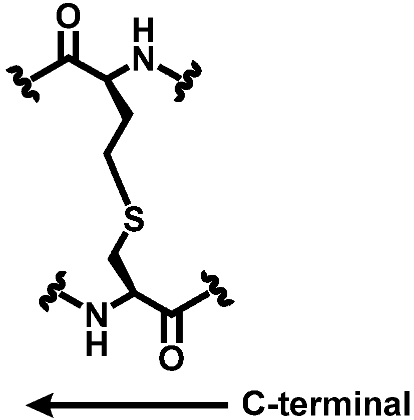
[CH₂,S] Cysthionine 1 (Ctt1) or gamma-cystathionine
   and 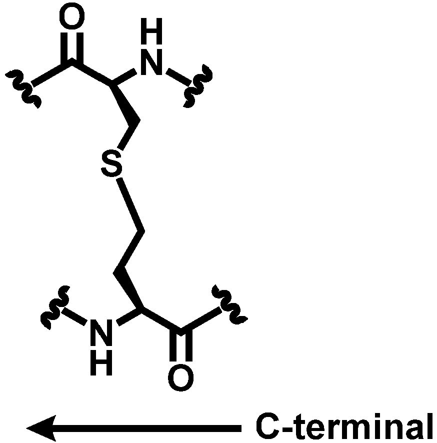
[S, CH₂] Cysthionine 2 (Ctt2) or delta-cystathionine

For ease of reference, it may be convenient to refer to the residues nominally present at positions X2 and X12 "before" formation of a thioether.

A cystathionine bridge may be considered to consist of the sulphur atom of a homocysteine side chain covalently linked to the beta-carbon atom (i.e. the side chain carbon atom) of an alanine residue.

Alternatively, a cystathionine bridge may be considered to be the product of a condensation reaction between homocysteine and serine to form a thioether bond.

In some compounds described herein therefore:
X2 is homocysteine (hC) and X12 is alanine (A) (designated cystathionine 1 (Ctt1) or gamma-cystathionine)
   or
X2 is alanine (A) and X12 is homocysteine (hC) (designated cystathionine 2 (Ctt2) or delta-cystathionine).

The notation used should not be taken to imply any particular method of synthesis.

Alternatively, the side chains of the residues at X2 and X12 may together represent a lanthionine (3,3-thiodialanine) bridge:

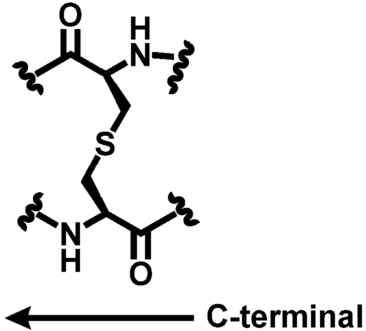

A lanthionine bridge may be considered to consist of the sulphur atom of a cysteine side chain covalently linked to the beta-carbon atom (i.e. the side chain carbon atom) of an alanine residue. Alternatively, it could be considered to be the product of a condensation reaction between cysteine and serine.

Thus, it may be convenient to refer to the residues nominally present at X2 and X12 as cysteine and alanine.

In the context of peptides of the invention, a lanthionine bridge is symmetrical and can have only one orientation, so it is irrelevant which residue is considered to be present at each position.

(Still further alternatively, a lanthionine bridge may be considered to be a thioether dimer of cysteine, so the residues at X2 and X12 could each be designated as cysteine. However, it will be understood that they are linked by a thioether bond rather than a disulfide bond.)

Thus, in some embodimentscompounds described herein
X2 is homocysteine and X12 is alanine (Ctt 1; gamma-cystathionine);
X2 is alanine and X12 is homocysteine (Ctt 2; delta-cystathionine); or
X2 is cysteine and X12 is alanine.

In addition to compounds having thioether bonds between the residues at positions X2 and X12, the examples describe molecules having similar peptide backbone sequences and acylations to the compounds of the invention, but containing cysteine residues at positions X2 and X12 linked by a disulfide bond.

In formulae using single letter amino acid notation in this specification, residues whose side chains participate in covalent bond are indicated by "(1)".

Thus, for example:
the sequence IC(1)IWQDWGAHRC(1)T contains a disulfide bond;
the sequence IhC(1)IWQDWGAHRA(1)T contains a Ctt 1 (gamma-cystathionine) bridge;
the sequence IA(1)IWQDWGAHRhC(1)T contains a Ctt 2 (delta-cystathionine) bridge;
the sequences IC(1)IWQDWGAHRA(1)T and IA(1)IWQDWGAHRC(1)T both contain a lanthionine bridge and would represent the same compound;

The terminal groups present at the N- and C-termini of the peptide backbone may be designated Y1 and Y2 respectively. Thus Y1 is bonded to the nitrogen atom of the N-terminal amino group and Y2 is bonded to the C-terminal carbonyl carbon atom.

Y1 = hydrogen (also indicated as "H-" or "Hy-") indicates a hydrogen atom, corresponding to the presence of a free primary or secondary amino group at the N-terminus. Y1 = acetyl ("Ac") indicates the presence of an N-terminal secondary acetyl amide group.

Y2 = "OH" or "NH₂" indicates the presence of a carboxy (COOH) group or an amido (CONH₂) group at the C-terminus of the molecule.

Either or both of Y1 and Y2 may alternatively be a lipophilic group Φ. Typicaly, only one of Y1 or Y2 will be a lipophilic group Φ.

In some embodiments, whether or not the molecule comprises a lipophilic group elsewhere, Y2 is NH₂ or OH. In some embodiments, Y1 is hydrogen or acetyl, and Y2 is OH or NH₂.

In some embodiments, whether or not the molecule comprises a lipophilic group elsewhere, Y2 is NH₂. In some embodiments, Y1 is hydrogen or acetyl, and Y2 is NH₂.

In some embodiments, whether or not the molecule comprises a lipophilic group elsewhere, Y2 is NH₂ and Y1 is acetyl.

Various terms relating to the methods and other aspects of the present invention are used throughout the specification and claims. Such terms are to be given their ordinary meaning in the art unless otherwise indicated. Other specifically defined terms are to be construed in a manner consistent with the definition provided herein. The term "about" as used herein when referring to a measurable value such as an amount, a temporal duration, and the like, is meant to encompass variations of ±20% or ±10%, in some embodiments ±5%, in some embodiments ±1%, and in some embodiments ±0.1% from the specified value, as such variations are appropriate to make and used the disclosed compounds and compositions.

The term "full length compstatin" as used herein refers to a 27 amino acid peptide having the sequence IC(1)VVQDWGHHRC(1)TAGHMANLTSHASAI, wherein C(1) denotes the cysteine residue linked by a disulfide bond. As described above, a truncated form of full length compstatin, the tridecapeptide Ile¹-Cys²-Val³-Val⁴-Gln⁵-Asp⁶-Trp⁷-Gly⁸-His⁹-His¹⁰-Arg¹¹-Cys¹²-Thr¹³-NH₂ linked by a disulfide bond between the cysteine residues at positions 2 and 12 retains the activity of the full length peptide. An N-terminally acetylated version of this tridecapeptide peptide is referred to herein as "Ac-compstatin".

The term "compstatin analogue" refers to a modified Ac-compstatin comprising one or more substitutions of natural and unnatural amino acids, or amino acid analogs, as well as modifications within or between various amino acids, as described in greater detail herein. A compstatin analogue may comprise about 1, 2, 3, 4 or 5 amino acid modifications relative to Ac-compstatin. A compstatin analogue may comprise 5, 6, 7, 8 or more amino acid modifications relative to Ac-compstatin. A compstatin analogue may comprise about 5, 6, 7 or 8 amino acid modifications relative to Ac-compstatin.

The term "analogue" is frequently used for a protein or peptide in question before it undergoes further chemical modification (derivatisation), and in particular acylation. The product resulting from such a chemical modification (derivatisation) is sometimes referred to as a "derivative" or "acylated analogue". However, in the context of this application, the term "analogue" designates analogues of Ac-compstatin as well as (the acylated) derivatives of such Ac-compstatin analogues.

When referring to the position of amino acids or analogs within Ac-compstatin or compstatin analogs, the positions are numbered from 1 (Ile in compstatin) to 13 (Thr in compstatin). For example, the Gly residue occupies "position 8." As used to describe the compstatin analogue peptides of the present invention "C(1)" denotes a disulfide bond between the respective cysteine residues in the compstatin analogue.

The terms "pharmaceutically active" and "biologically active" refer to the ability of compounds to bind C3 or fragments thereof and inhibit complement activation. The biological activities of compstatin analogs may be measured by one or more of several art-recognized assays, as described in greater detail herein.

As used herein, "L-amino acid" refers to any of the naturally occurring levorotatory alpha-amino acids normally present in proteins or the alkyl esters of those alpha-amino acids. The term "D-amino acid" refers to dextrorotatory alpha-amino acids. Unless specified otherwise, all amino acids referred to herein are L-amino acids.

"Hydrophobic" or "non-polar" are used synonymously herein, and refer to any inter- or intramolecular interaction not characterized by a dipole.

As used herein, "pharmaceutically-acceptable salts" refers to derivatives of the disclosed compounds wherein the parent compound is modified by making acid or base salts thereof. Examples of pharmaceutically-acceptable salts include, but are not limited to, mineral or organic acid salts of basic residues such as amines; alkali or organic salts of acidic residues such as carboxylic acids; and the like. Thus, the term "acid addition salt" refers to the corresponding salt derivative of a parent compound that has been prepared by the addition of an acid. The pharmaceutically-acceptable salts include the conventional salts or the quaternary ammonium salts of the parent compound formed, for example, from inorganic or organic acids. For example, such conventional salts include, but are not limited to, those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like. Certain acidic or basic compounds of the present invention may exist as zwitterions. All forms of the compounds, including free acid, free base, and zwitterions, are contemplated to be within the scope of the present invention.

### Compstatin Analogues

Ac-Compstatin, an N-terminally acetylated 13 amino acid peptide, is known to bind to C3 and prevent C3 convertase-mediated cleavage. Since its discovery by phage display, modification to the 13 amino acid Ac-Compstatin sequence has been carried out in an effort to find analogues with increased biological activity. However, in the core sequence between the two cysteines residues at positions 2 and 12, alanine scanning experiments have previously produced analogues showing only modest improvements in biological activity, with few modifications being tolerated. The modifications include changing the valine at position 4 to tryptophan, or a tryptophan analogue, that leads to an increase in biological activity and changing the histidine at position 9 to alanine or analogs thereof.

In particular, previous attempts to introduce modifications to the valine residue at position 3, replacing it with glycine, alanine, D-valine or leucine have been shown to lead to a decrease in biological activity. In contrast to these prior art findings, the present inventors surprisingly found that a change of valine to isoleucine is well tolerated and provides improvements in biological activity, as shown in the Examples below.

Without wishing to be bound by any specific theory, the present inventors reasoned that this modification might be combined with introduction of one or more polar or charged amino acids in the core sequence and may be used as an approach to increase the ability of the compstatin peptides to solubilize. Initially, glutamic acid or serine at position 9 were combined with valine 3 and led to a decrease in activity compared to the reference sequence 4W9A. However, when these changes were combined with the introduction of isoleucine at position 3, a surprising increase in biological activity was observed, in particular for the combination of isoleucine at position 3 and glutamic acid at position 9. This observation correlates with improved binding to C3 as measured by surface plasmon resonance (SPR), see Table 7 and Table 8.

In a further series of experiments to validate these findings, compstatin peptides with glutamic acid at position 9 are combined with different substitutions in position 3 which would normally be considered "conservative" replacements for isoleucine, again showing that the peptides with isoleucine at position 3 are most active.

Taken together, these experiments show that replacing the valine residue at position 3 with isoleucine surprisingly provides compstatin peptides having increased biological activity and improved binding to C3. Furthermore, the experiments surprisingly demonstrate that these changes can be readily combined with other modifications in the core sequence of the compstatin analogues and with addition of N and C-terminal sequences, for example for improving the solubility of the compstatin peptides, e.g. at higher concentrations.

Introduction of isoleucine instead of valine at position 3 of a further prior art compound designated "Cp40" (Qu et al., Immunobiology 2013, 281(4): 496-505; also referred to in that paper as "peptide 14) also increased the binding affinity to C3 as measured by SPR.

### Lipophilic substituents

The compstatin analogues bear a lipophilic group, which may be designated Φ.

The lipophilic group is covalently linked to the side chain of the C-terminal Lys residue of the analogue.

The lipophilic group Φ is typically attached via an acyl group. The modification may therefore be termed acylation but can also be refered to as lipidation.

The lipophilic group includes a long chain alkylene group derived from a fatty acid, termed Z¹ herein and referred to as the lipophilic substituent. Without wishing to be bound by theory, it is believed that a lipophilic substituent binds plasma proteins (e.g. albumin) in the blood stream, thus shielding the compounds employed in the context of the invention from enzymatic degradation, and thereby enhancing the half-life of the compounds. The lipophilic substituent may also modulate the potency of the compound.

Z¹ may be attached directly to the amino acid sequence or via a spacer Z² as defined herein.

In other words, Φ may be Z¹- or Z¹-Z²-.

The term "conjugated" is used here to describe the covalent attachment of one identifiable chemical moiety to another, and the structural relationship between such moieties. It should not be taken to imply any particular method of synthesis. The one or more spacers Z², when present, are used to provide a spacing between the compound and the lipophilic substituent Z¹.

Z¹ is COOH-C₁₂₋₂₂alkylene-(CO)-, wherein the akylene may be linear or branched and may be saturated or unsaturated, and may optionally incorporate a phenylene or piperazinylene moiety in its length.

Typically, Z¹ groups are derived from long-chain saturated α,ω-dicarboxylic acids of formula HOOC-(CH₂)₁₂₋₂₂-COOH, preferably from long-chain saturated α,ω-dicarboxylic acids having an even number of carbon atoms in the aliphatic chain.

For example, Z¹ may be:
13-carboxytridecanoyl, i.e. HOOC-(CH₂)₁₂-(CO)-;
15-carboxypentadecanoyl, i.e. HOOC-(CH₂)₁₄-(CO)-;
17-carboxyheptadecanoyl, i.e. HOOC-(CH₂)₁₆-(CO)-;
19-carboxynonadecanoyl, i.e. HOOC-(CH₂)₁₈-(CO)-; or
21-carboxyheneicosanoyl, i.e. HOOC-(CH₂)₂₀-(CO)-

As mentioned above, the lipophilic substituent Z¹ may be conjugated to the amino acid side chain by one or more spacers Z².

When present, the spacer is attached to the lipophilic substituent and to the amino acid side chain.

Z² is selected from:
[γGlu];
[γGlu][Peg3][Peg3]-;
[(Piperazine-1-yl)-acetyl][Peg3][Peg3];
[γGlu]-G-[γGlu];
[γGlu]-K-[γGlu];
[yGlu]-KG-[yGlu]; and
[γGlu]-G-[Peg3][γGlu][Peg3].

Z² is suitably bound at each side by amide linkage. Other suitable linkages may be used, with the commensurate atom replacement; for example sulfinamide, sulfonamide, or ester linkages or amino, ether, or thioether linkages are envisaged.

Z¹ groups derived from long-chain saturated α,ω-dicarboxylic acids of formula HOOC-(CH₂)₁₂₋₂₂-COOH are described and illustrated below

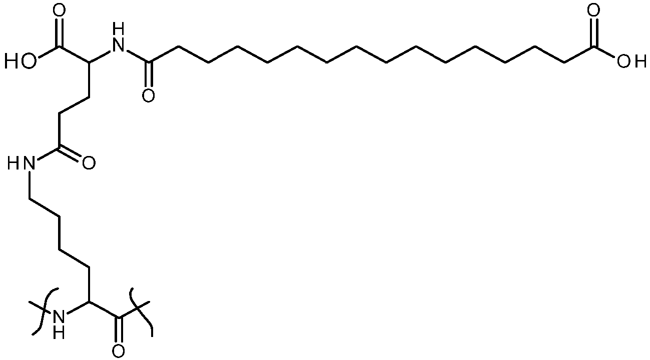

Here, the side chain of a Lys residue is covalently attached to a yGlu spacer (Z²) via an amide linkage. A 15-carboxypentadecanoyl group (Z¹) is covalently attached to the yGlu spacer via an amide linkage.This combination of lipophilic moiety and spacer, conjugated to a Lys residue, may be referred to by the short-hand notation K(15-carboxypentadecanoyl-γ-Glu), e.g., when shown in formulae of specific compounds. yGlu can also be referred to as isoGlu.

Certain preferred Φ groups (Z¹- and Z¹-Z²-) include:
[15-carboxy-pentadecanoyl];
[15-carboxy-pentadecanoyl][yGlu],
[15-carboxy-pentadecanoyl][γGlu][Peg3][Peg3];
[19-carboxy-nonadecanoyl][γGlu][Peg3][Peg3];
[15-carboxy-pentadecanoyl][(Piperazine-1-yl)-acetyl][Peg3][Peg3];
[17-carboxy-heptadecanoyl][γGlu]G[γGlu];
[17-carboxy-heptadecanoyl][γGlu]K[γGlu];
[17-carboxy-heptadecanoyl][γGlu]KG[γGlu];
[17-carboxy-heptadecanoyl][γGlu]G[Peg3][γGlu][Peg3];
[15-carboxy-pentadecanoyl][γGlu]G[γGlu];
[17-carboxy-heptadecanoyl];
[17-carboxy-heptadecanoyl][γGlu]
[19-carboxy-nonadecanoyl][γGlu]G[γGlu];and
[17-carboxy-heptadecanoyl][γGlu][Peg3][Peg3].

Illustrative structures of Φ groups (Z¹- and Z¹-Z²- ) are shown below, where the wavy line indicates the linkage to the peptide (to an amino acid side chain, N-terminal nitrogen, or C-terminal carbon):
[19-carboxy-nonadecanoyl][yGlu]G[yGlu]: 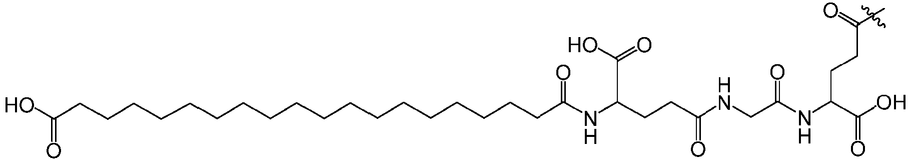
[17-carboxy-heptadecanoyl][γGlu]G[γGlu]: 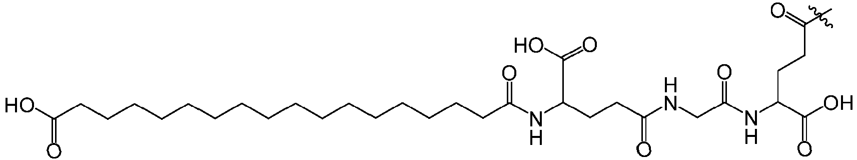
[15-carboxy-pentadecanoyl]- : 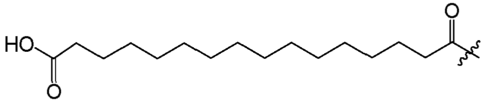
[17-carboxy-heptadecanoyl]- : 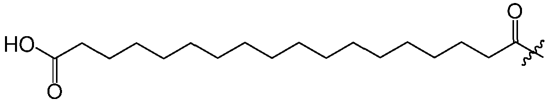
[(15-carboxy-pentadecanoyl)-[(Piperazine-1-yl)-acetyl][Peg3][Peg3]: 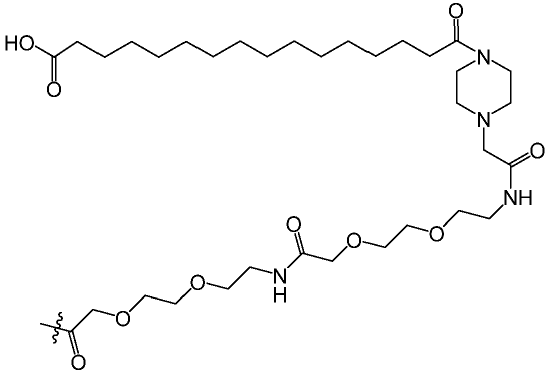
[17-carboxy-heptadecanoyl][γGlu]: 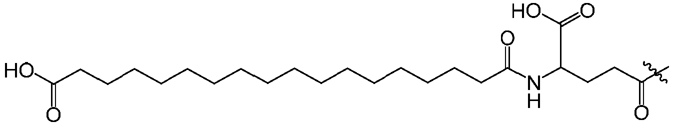
[17-carboxy-heptadecanoyl][γGlu]G[Peg3][γGlu][Peg3]: 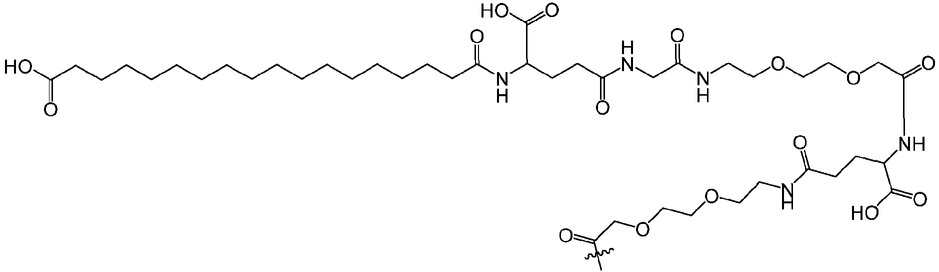
[17-carboxy-heptadecanoyl][γGlu]KG[γGlu]: 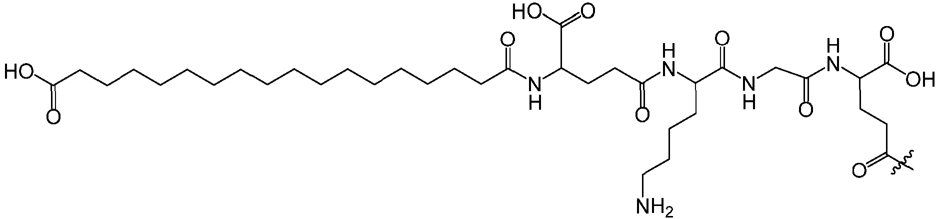
[17-carboxy-heptadecanoyl][γGlu]K[γGlu]: 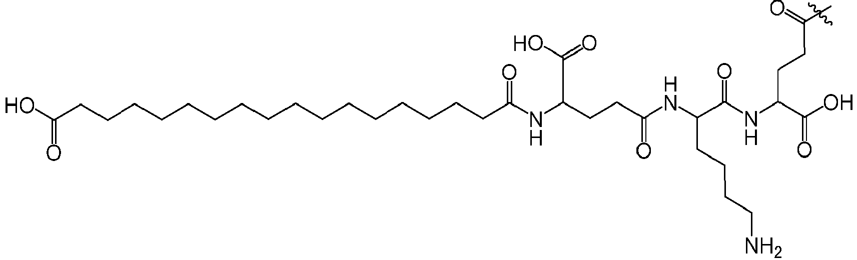
[17-carboxy-heptadecanoyl][γGlu][Peg3][Peg3]:

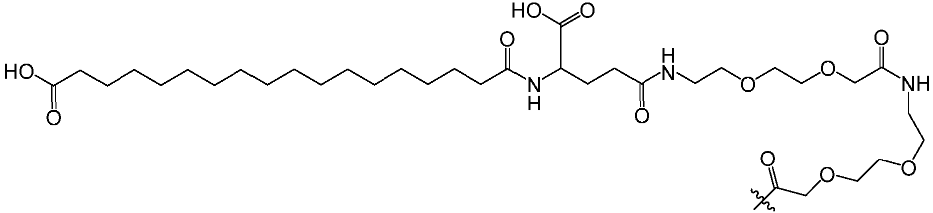

The skilled person will be well aware of suitable techniques for preparing the compounds employed in the context of the invention. For examples of suitable chemistry, see WO98/08871, WO00/55184, WO00/55119, Madsen et al., J. Med. Chem. 50:6126-32 (2007), and Knudsen et al., J. Med Chem. 43:1664-1669 (2000).

Compstatin analogues made in the prior art have been shown to possess improved activity as compared with the parent peptide, i.e., up to about 99-fold (Mallik, B. et al, 2005, supra; WO 2004/026328), and up to about 264-fold (Katragadda et al., 2006, supra; WO2007/062249).

In accordance with the present invention, information about the biological and physicochemical characteristics of Ac-compstatin binding to C3 have been employed to design compstatin analogues with significantly improved activity compared to the parent compstatin analogues.

Preferably, the compstatin analogs have greater activity than Ac-compstatin, e.g. at least 10-fold greater activity, at least 20-fold greater activity, at least 30-fold greater activity than Ac-compstatin. In other embodiments, the analogs have at least 40-, 50-, 60-, 70-, 80-, 90-, 100-, 110-, 120-, 130-, 140-, 150-fold or greater activity than Ac-compstatin, as compared utilizing the assays described in the examples.

A compound of the invention typically has greater activity than an otherwise identical compound having valine instead of isoleucine at the position corresponding to Val3 of compstatin.

The compstatin analogues are capable of binding to C3 and/or C3b, and of inhbiting activation of the complement cascade, particularly downstream of C3, e.g. by inhibiting cleavage of C3 by C3 convertases.

The compstatin analogues are also typically capable of inhibiting complement-driven haemolysis. Complement-driven haemolysis is typically assessed (in a "haemolysis assay") by contacting serum from a first mammalian species (e.g. human serum) with erythrocytes (red blood cells; RBC) from a second mammalian species (e.g. sheep or any other suitable species), typically in the presence of mammalian immunoglobulin capable of binding to the erythrocytes. Complement in the serum is activated by the cell-bound immunoglobulin, leading to lysis of the erythrocytes, i.e. haemolysis. The immunoglobulin may be from the first species, or may be from a third mammalian species as long as it is capable of activating complement from the first species.

In such an assay, a test compound will typically be pre-incubated with the serum before the serum is contacted with the erythroctes. The erythrocytes may also be pre-incubated with the immunoglobulin before contacting with the serum.

In the examples below, human serum is pre-incubated with a test compound, and sheep erythroctes are pre-incubated with rabbit anti-serum against sheep erythrocytes, before the serum and erythrocytes are combined.

Thus, the activity of the compstatin analogues may be determined with reference to one or more biological activities selected from (1) binding to C3 protein, (2) binding to C3b protein, (3) inhibiting the cleavage of native C3 by C3 convertases, and (4) inhibiting the activation of the complement system.

Thus a compstatin analogue of the invention may bind C3 or C3b with a higher affinity than that of compstatin. For example, they may have a Kd at least 10-fold lower, at least 20-fold lower, or at least 30-fold lower than Ac-compstatin, e.g. at least 40-, 50-, 60-, 70-, 80-, 90-, 100-, 110-, 120-, 130-, 140-, or 150-fold lower than Ac-compstatin. The Kd may be determined by surface plasmon resonance (SPR), e.g. using an assay as described in Example 4.

A compstatin analogue of the invention typically binds C3 or C3b with a greater affinity (i.e. a lower Kd) than that of an otherwise identical compound having valine instead of isoleucine at the position corresponding to Val3 of compstatin.

A compstatin analogue of the invention may have a greater ability to inhibit haemolysis than Ac-compstatin. For example, it may inhibit haemolysis with an IC₅₀ at least 10-fold, at least 20-fold, or at least 30-fold lower than Ac-compstatin, e.g. at least 40-, 50-, 60-, 70-, 80-, 90-, 100-, 110-, 120-, 130-, 140-, 150-, 200-, 250-, 300- 350-, 400-, 450-, 500-fold lower than Ac-compstatin.

A compstatin analogue of the invention typically has a greater ability to inhibit haemolysis (i.e. a lower IC₅₀) than an otherwise identical compound having valine instead of isoleucine at the position corresponding to Val3 of compstatin.

Preferably, the *in vitro* effect of the compounds of the present invention are assessed by measuring their inhibitory effect on the classical complement pathway in a haemolysis assay, e.g. using the assay described in Example 2.

Compstatin analogues having acylation may have a lower absolute activity than an otherwise identical compound lacking acylation, but have additional benefits including prolonged in vivo half life which may offset any apparent reduction of absolute activity.

### Synthesis of Compstatin Analogues

It is preferred to synthesize compstatin analogues of the present invention by means of solid-phase or liquid-phase peptide synthesis methodology. In this context, reference may be made to WO 98/11125 and, among many others, Fields, G.B. et al., 2002, "Principles and practice of solid-phase peptide synthesis". In: Synthetic Peptides (2nd Edition), and the Examples herein.

Details regarding the synthesis and structures of compstatin analogues containing cystathionine and lanthionine bridges between the residues at positions X2 and X12 are provided in WO2012/040259, and in Knerr et al., ACS Chem Biol. 2011 July 15; 6(7): 753-760 (DOI:10.1021/cb2000378). Further relevant details regarding cystathionine and lanthionine chemistry can be found in de Araujo et al., 2014, Nature Communications; 5:3165 (DOI: 10.1038/ncomms4165) and in Muttenthaler et al., J. Med. Chem. 2010, 53, 8585-8596 (DOI: 10.1021/jm100989w).

In accordance with the present invention, a compstatin analogue of the invention may be synthesized or produced in a number of ways, including for example, a method which comprises:
(a) synthesizing the compstatin analogues by means of solid-phase or liquid-phase peptide synthesis methodology and recovering the synthesized compstatin analogues thus obtained; or
(b) expressing a precursor peptide sequence from a nucleic acid construct that encodes the precursor peptide, recovering the expression product, and modifying the precursor peptide to yield a compound of the invention.

The precursor peptide may be modified by introduction of one or more non-proteinogenic amino acids, e.g. Aib, Orn, Dap, 1-Me-Trp, 1-Nal, 2-Nal, Sar, yGlu or Dab, or by the introduction of an appropriate terminal groups Y1 and/or Y2.

Expression is typically performed from a nucleic acid encoding the precursor peptide, which may be performed in a cell or a cell-free expression system comprising such a nucleic acid.

It is preferred to synthesize the analogues of the invention by means of solid-phase or liquid-phase peptide synthesis. In this context, reference is made to WO 98/11125 and, among many others, Fields, GB et al., 2002, "Principles and practice of solid-phase peptide synthesis". In: Synthetic Peptides (2nd Edition), and the Examples herein.

For recombinant expression, the nucleic acid fragments encoding the precursor peptide will normally be inserted in suitable vectors to form cloning or expression vectors. The vectors can, depending on purpose and type of application, be in the form of plasmids, phages, cosmids, mini-chromosomes, or virus, but also naked DNA which is only expressed transiently in certain cells is an important vector. Preferred cloning and expression vectors (plasmid vectors) are capable of autonomous replication, thereby enabling high copy-numbers for the purposes of high-level expression or high-level replication for subsequent cloning.

In general outline, an expression vector comprises the following features in the 5'→3' direction and in operable linkage: a promoter for driving expression of the nucleic acid fragment, optionally a nucleic acid sequence encoding a leader peptide enabling secretion (to the extracellular phase or, where applicable, into the periplasma), the nucleic acid fragment encoding the precursor peptide, and optionally a nucleic acid sequence encoding a terminator. They may comprise additional features such as selectable markers and origins of replication. When operating with expression vectors in producer strains or cell lines it may be preferred that the vector is capable of integrating into the host cell genome. The skilled person is very familiar with suitable vectors and is able to design one according to their specific requirements.

The vectors of the invention are used to transform host cells to produce the precursor peptide. Such transformed cells can be cultured cells or cell lines used for propagation of the nucleic acid fragments and vectors, and/or used for recombinant production of the precursor peptides.

Preferred transformed cells are micro-organisms such as bacteria [such as the species *Escherichia* (e.g. *E. coli*), *Bacillus* (e.g. *Bacillus subtilis*), Salmonella, or *Mycobacterium* (preferably non-pathogenic, e.g. *M. bovis* BCG), yeasts (e.g., Saccharomyces cerevisiae and Pichia pastoris), and protozoans. Alternatively, the transformed cells may be derived from a multicellular organism, i.e. it may be fungal cell, an insect cell, an algal cell, a plant cell, or an animal cell such as a mammalian cell. For the purposes of cloning and/or optimised expression it is preferred that the transformed cell is capable of replicating the nucleic acid fragment of the invention. Cells expressing the nucleic fragment can be used for small-scale or large-scale preparation of the peptides of the invention.

When producing the precursor peptide by means of transformed cells, it is convenient, although far from essential, that the expression product is secreted into the culture medium.

### Medical Conditions

In a broad aspect, the present invention provides compstatin analogues of the present invention for use as a medicament or for use in therapy.

The compstatin analogues described herein have biological activities of binding to C3 protein and/or inhibiting complement activation. Generally, the compstatin analogues of the present invention may be used for the treatment or prevention conditions associated with excessive or unwanted activation of the complement system. Complement can be activated through three different pathways: the classical, lectin and alternative pathways. The major activation event that is shared by all three pathways is the proteolytic cleavage of the central protein of the complement system, C3, into its activation products C3a and C3b by C3 convertases. Generation of these fragments leads to the opsonization of pathogenic cells by C3b and iC3b, a process that renders them susceptible to phagocytosis or clearance, and to the activation of immune cells through an interaction with complement receptors (Markiewski & Lambris, 2007, Am. J. Pathol., 171: 715-727). Deposition of C3b on target cells also induces the formation of new convertase complexes and thereby initiates a self-amplification loop. An ensemble of plasma and cell surface-bound proteins carefully regulates complement activation to prevent host cells from self-attack by the complement cascade. The 13 amino acid cyclic tridecapeptide used as a reference point for the design of the compstatin analogues of the present invention inhibits complement activation by binding to C3 and/or C3b, preventing the cleavage of native C3 by the C3 convertases. Without wishing to be bound by any particular theory, the present inventors believe that the compstatin analogues of the present invention also function in this way and may share one or more biological activities selected from (1) binding to C3 protein, (2) binding to C3b protein, (3) inhibiting the cleavage of native C3 by C3 convertases, and/or (4) inhibiting the activation of the complement system. The biological activity of the compstatin analogues of the present invention may be determined *in vitro by* measuring their inhibitory effect of the classical complement pathway in a haemolysis assay, for example using a protocol set out in the examples below.

Excessive activation or inappropriate regulation of complement can lead to a number of pathologic conditions, ranging from autoimmune diseases to inflammatory diseases (Holers, 2003, Clin. Immunol., 107: 140-51; Markiewski & Lambris, 2007, supra; Ricklin & Lambris, 2007, Nat. Biotechnol., 25: 1265-75; Sahu et al., 2000, J. Immunol., 165: 2491-9). Inhibition of complement activation may thus be used to facilitate treatment of diseases or conditions including age-related macular degeneration, Stargardt disease, periodontitis, diabetic retinopathy, glaucoma, uveitis, rheumatoid arthritis, spinal cord injury, stroke, multiple sclerosis, Parkinson's disease, Alzheimer's disease, cancer, and respiratory disorders such as asthma, chronic obstructive pulmonary disease (COPD), allergic inflammation, emphysema, bronchitis, bronchiecstasis, cystic fibrosis, tuberculosis, pneumonia, respiratory distress syndrome (RDS - neonatal and adult), rhinitis and sinusitis; bacterial infections such as sepsis, ischemia-reperfusion injury in various tissues, myocardial infarction, anaphylaxis, paroxysmal nocturnal hemoglobinuria, autoimmune hemolytic anemias, psoriasis, hidradentitis suppurativa, myasthenia gravis, systemic lupus erythematosus, CHAPLE syndrome, C3 glomeropathy, IgA nephropathy, atypical hemolytic uremic syndrome, Crohn's disease, ulcerative colitis and antiphospholipid syndrome. The analogue of the invention may also find use in) inhibiting complement activation that occurs during cell or solid organ transplantation, or in the use of artificial organs or implants (e.g., by coating or otherwise treating the cells, organs, artificial organs or implants with a peptide of the invention); or inhibiting complement activation that occurs during extracorporeal shunting of physiological fluids (blood, urine) (e.g., by coating the tubing through which the fluids are shunted with a compstatin analogue of the present invention).

### Pharmaceutical Compositions and Administration

In a further aspect, the present invention relates to a composition comprising a compstatin analogue according to the invention, or a pharmaceutically acceptable salt or solvate thereof, together with a carrier. In one embodiment of the invention, the composition is a pharmaceutical composition and the carrier is a pharmaceutically acceptable carrier. The present invention also relates to a pharmaceutical composition comprising a compstatin analogue according to the invention, or a salt and/or solvate thereof, together with a carrier, excipient or vehicle. Accordingly, the compstatin analogue of the present invention, or salts or solvates thereof, especially pharmaceutically acceptable salts and/or solvates thereof, may be formulated as compositions or pharmaceutical compositions prepared for storage or administration, and which comprise a therapeutically effective amount of a compstatin analogue of the present invention, or a salt or solvate thereof.

Suitable salts formed with bases include metal salts, such as alkali metal or alkaline earth metal salts.

In one embodiment, a pharmaceutical composition of the invention is one wherein the compstatin analogue is in the form of a pharmaceutically acceptable acid addition salt.

As will be apparent to one skilled in the medical art, a "therapeutically effective amount" of a compstatin analogue compound or pharmaceutical composition thereof of the present invention will vary depending upon, inter alia, the age, weight and/or gender of the subject (patient) to be treated. Other factors that may be of relevance include the physical characteristics of the specific patient under consideration, the patient's diet, the nature of any concurrent medication, the particular compound(s) employed, the particular mode of administration, the desired pharmacological effect(s) and the particular therapeutic indication. Because these factors and their relationship in determining this amount are well known in the medical arts, the determination of therapeutically effective dosage levels, the amount necessary to achieve the desired result of treating and/or preventing and/or remedying malabsorption and/or low-grade inflammation described herein, as well as other medical indications disclosed herein, will be within the ambit of the skilled person.

As used herein, the term "a therapeutically effective amount" refers to an amount which reduces symptoms of a given condition or pathology, and preferably which normalizes physiological responses in an individual with that condition or pathology. Reduction of symptoms or normalization of physiological responses can be determined using methods routine in the art and may vary with a given condition or pathology. In one aspect, a therapeutically effective amount of one or more compstatin analogues, or pharmaceutical compositions thereof, is an amount which restores a measurable physiological parameter to substantially the same value (preferably to within 30%, more preferably to within 20%, and still more preferably to within 10% of the value) of the parameter in an individual without the condition or pathology in question.

In one embodiment of the invention, administration of a compound or pharmaceutical composition of the present invention is commenced at lower dosage levels, with dosage levels being increased until the desired effect of preventing/treating the relevant medical indication is achieved. This would define a therapeutically effective amount. For the compstatin analogues of the present invention, alone or as part of a pharmaceutical composition, such human doses of the active compstatin analogue may be between about 0.01 pmol/kg and 500 µmol/kg body weight, between about 0.01 pmol/kg and 300 µmol/kg body weight, between 0.01 pmol/kg and 100 µmol/kg body weight, between 0.1 pmol/kg and 50 µmol/kg body weight, between 1 pmol/kg and 10 µmol/kg body weight, between 5 pmol/kg and 5 µmol/kg body weight, between 10 pmol/kg and 1 µmol/kg body weight, between 50 pmol/kg and 0.1 µmol/kg body weight, between 100 pmol/kg and 0.01 µmol/kg body weight, between 0.001 µmol/kg and 0.5 µmol/kg body weight, between 0.05 µmol/kg and 0.1 µmol/kg body weight.

The therapeutic dosing and regimen most appropriate for patient treatment will of course vary with the disease or condition to be treated, and according to the patient's weight and other parameters. Without wishing to be bound by any particular theory, it is expected that doses, in the mg/kg range, and shorter or longer duration or frequency of treatment may produce therapeutically useful results, such as a statistically significant inhibition of the alternative and classical complement pathways. The dosage sizes and dosing regimen most appropriate for human use may be guided by the results obtained by the present invention, and may be confirmed in properly designed clinical trials.

An effective dosage and treatment protocol may be determined by conventional means, starting with a low dose in laboratory animals and then increasing the dosage while monitoring the effects, and systematically varying the dosage regimen as well. Numerous factors may be taken into consideration by a clinician when determining an optimal dosage for a given subject.

For local delivery to the eye, the pharmaceutically acceptable compositions may be formulated in isotonic, pH adjusted sterile saline or water, either with or without a preservative such as benzylalkonium chloride. Alternatively, for ophthalmic uses, the pharmaceutically acceptable compositions may be formulated in an ointment such as petrolatum or as eyedrops. Methods of local administration to the eye include, e.g., choroidal injection, transscleral injection or placing a scleral patch, selective arterial catheterization, eyedrops or eye ointments, intraocular administration including transretinal , subconjunctival bulbar, intravitreous injection, suprachoroidal injection, subtenon injection, scleral pocket and scleral cutdown injection, by osmotic pump, etc. The agent can also be alternatively administered intravascularly, such as intravenously (IV) or intraarterially. In choroidal injection and scleral patching, the clinician uses a local approach to the eye after initiation of appropriate anesthesia, including painkillers and ophthalmoplegics. A needle containing the therapeutic compound is directed into the subject's choroid or sclera and inserted under sterile conditions. When the needle is properly positioned the compound is injected into either or both of the choroid or sclera. When using either of these methods, the clinician can choose a sustained release or longer acting formulation. Thus, the procedure can be repeated only every several months or several years, depending on the subject's tolerance of the treatment and response.

The following examples are provided to describe the invention in greater detail. They are intended to illustrate, not to limit, the invention. The compounds described have particularly advantageous properties as a result of their particular amino acid sequences and/or acylation. In addition to compounds having residues linked by thioether bonds at the positions corresponding to positions 2 and 12 of compstatin, certain of the compounds described below have cysteine residues linked by disulfide bonds at those positions. It is believed that similar or otherwise identical compounds containing thioether linkages will have similar advantageous properties, and/or will show improvements in stability, such as chemical stability (resistance to degradation) or physical stability (resistance to aggregation). However, only compounds covered by the claims are compounds of the invention,

### Example 1: Synthesis of Compstatin Analogues

### General Peptide Synthesis

| **List of abbreviations and suppliers** | | | | |
|---|---|---|---|---|
| | **Abbreviation** | **Name** | **Brand / Supplier** | |
| **Resins** | | | | |
| | | TentaGel^{™} PHB AA(Proct)-Fmoc | Rapp Polymere | |
| | | TentaGel^{™} SRAM | Rapp Polymere | |
| | | Rink amide-MBHA | - | |
| **Amino acids** | | | | |
| | | Pseudoprolines (E.g. YS, FS, FT) | Jupiter Bioscience Ltd. | |
| | | Fmoc-L-Aaa-OH | Senn Chemicals AG | |
| **Coupling reagents** | | | | |
| | Oxyma Pure | Ethyl cyanoglyoxylate-2-oxime | Chem Impex international | |
| | DIC | Diisopropylcarbodiimide | Fluka / Sigma Aldrich Co. | |
| | HATU | N-[(dimethylamino)-1H-1,2,3-triazol[4,5-b]pyridine-1-ylmethylene]-N-methylmethanaminium hexafluorophosphate N-oxide | ChemPep Inc. | |
| | HOBt | Hydroxybenzotriazole | Sigma-Aldrich Co. | |
| | HBTU | | - | |
| | PyAOP | | | |

| **Solvents and reagents** | | | | |
|---|---|---|---|---|
| | Boc₂O | Di-tert-butyl pyrocarbonate | Advanced ChemTech | |
| | DCM | Dichloromethane | Prolabo (VWR) | |
| | DIPEA | Diisopropylethylamine | Fluka / Sigma Aldrich Co. | |
| | NMM | | - | |
| | DMF | N,N-dimethylformamide | Taminco | |
| | Et₂O | Diethyl ether | Prolabo (VWR) | |
| | EtOH | Ethanol | CCS Healthcare AB | |
| | HCOOH | Formic acid (HPLC grade) | Sigma-Aldrich Co. | |
| | H₂O | Water, Milli-Q water | Millipore | |
| | MeCN | Acetonitrile (HPLC) | Sigma-Aldrich Co. | |
| | NMP | N-methylpyrrolidone | Sigma-Aldrich Co. | |
| | MeOH | Methanol | Sigma-Aldrich Co | |
| | | Piperidine | Jubliant Life Sciences Ltd. | |
| | TFA | Trifluoroacetic acid (HPLC) | Chemicals Raw Materials Ltd. | |
| | TIS | Triisopropylsilane | Sigma-Aldrich Co. | |
| | DODT | 2,2'-(ethylenedioxy)diethanethiol | Sigma-Aldrich Co. | |
| Other reagents | | Ascorbic acid | Sigma-Aldrich Co. | |
| | I₂ | Iodine | Sigma-Aldrich Co | |
| | | | | |
| | Pd[P(C₆H₅)₃]₄ | Tetrakis(triphenylphosphine) palladium | Sigma-Aldrich Co. | |
| | N₂H₄ | Hydrazine | Sigma-Aldrich Co | |
| | | | | |

### Apparatus and synthetic strategy

Peptides were synthesized batch wise manually or on a peptide synthesiser, such as a CEM Liberty Peptide Synthesizer or a Symphony X Synthesizer, according to solid phase peptide synthetic procedures using 9-fluorenylmethyloxycarbonyl (Fmoc) as N-α-amino protecting group and suitable common protection groups for side-chain functionalities.

Polymeric support based resins, such as e.g. TentaGel^{™}, was used. The resin was swelled in DMF prior to initiation of the solid phase synthesis.

### Standard amino acid coupling procedures

### Manual peptide coupling

Manual coupling was performed with 2-3 equiv. of Fmoc-protected amino acid and either, HATU:NMM (2-3 : 4-6 equiv), HBTU:NMM (2-3 : 4-6 equiv), DIC:Oxyma (2-3 : 2-3) or DIC:Oxyma:DIPEA (2-3 : 2-3 : 0.3-6). Amino acid coupling times from 45 min to 1 hr while shaken followed by thorough washing.

### CEM Liberty Peptide Synthesizer

A solution of Fmoc-protected amino acid (4 equiv.) coupling reagent (4 equiv.) and base (8 equiv.) was added to the resin. The mixture was either heated by the microwave unit to 70-75°C for 5 min or coupled without heating for 60 min. During the coupling nitrogen was bubbled through the mixture.

### Symphony X Synthesizer

The amino acid coupling reagents were transferred to the reaction vessels in the following order: Fmoc-protected amino acid (4 equiv.), HATU (4 equiv.) and DIPEA (8 equiv.). The coupling time was 10 min at room temperature (rt.) unless otherwise stated. The resin was washed with DMF (5 x 0.5 min). In case of repeated couplings the coupling time was in all cases 45 mins at rt.

### Fmoc deprotection

### Manual Fmoc deprotection

Following a thorough washing of the resin a solution of 20 v/v/% piperazine in DMF was added and the mixture has left reacting for 30 min while shaken. Following the coupling, the resin was washed appropriately.

### CEM Liberty Peptide Synthesizer

The Fmoc group was deprotected using piperidine in DMF or other suitable solvents. The deprotection solution was added to the reaction vessel and the mixture was heated for 30 sec. reaching approx. 40°C. The reaction vessel was drained and fresh deprotection solution was added and subsequently heated to 70-75°C for 3 min. After draining the reaction vessel the resin was washed with DMF or other suitable solvents.

### Symphony X Synthesizer

Fmoc deprotection was performed for 2.5 min using 40% piperidine in DMF and repeated using the same conditions. The resin was washed with DMF (5 x 0.5 min).

### Side chain acylation

Fmoc-Lys(Dde)-OH or amino acid with an alternative orthogonal side chain protective group was introduced at the position of the acylation (side-chain lipidation). The N-terminal of the linear peptide was either acetylated or Boc protected. While the peptide was still attached to the resin, the protecting group of the lysine ^{ε}-amine side chain was selectively cleaved using freshly prepared hydrazine hydrate (2-4%) in NMP for 2 x 15 min. The unprotected lysine side-chain was then elongated using standard coupling conditions and Fmoc-deprotections with the desired building block according to the specific sequence. The lipid moiety was coupled as the last step.

Alternatively, the side chain acylation was assembled prior to assembly of the linear peptide. Dde-Lys(Fmoc)-OH or alternatively other orthogonal protection groups were introduced at the C-terminal at the position of the acylation (side-chain lipidation). The Fmoc group was then removed as described under the deprotection section and the unprotected lysine side-chain was elongated using standard amino acid coupling conditions and Fmoc-deprotections with the desired building block. The lipidation moiety was coupled as the last step. While the branched lysine was still attached to the resin, the orthogonal N-terminal protective group (Dde) was selectively cleaved using freshly prepared hydrazine hydrate (2-4%) in NMP for 2 x 15 min and the standard peptide synthesis continued.

### Incorporation of the Dde-hCys(Fmoc-Ala-OAllyl)-OH (#1) or Dde-Ala(Fmoc-hCys-OAllyl)-OH (#2)

1.5-3.0 equiv. of #1 or #2 (scheme 1a) was used and the building block was incorporated using standard coupling reagents and an extended coupling time of 2.5 hr to 12 hr.

### Allyl deprotection and formation of the lactam:

Following assembling of the branched peptide on-resin the OAII group was removed by treating the resin with a solution of Pd(P(C₆H₅)₃]₄ in CHCl₃/AcOH/NMM for 3 hours following a thorough wash with DMF. The Fmoc group was removed using standard deprotection conditions. The amide bond formedbetween the free amine and carboxylic acid was formed using PyAOP, HOBt and DIPEA (3:5:5) in DMF overnight.

### Release of peptide from the solid support

The dried peptide resin was treated with TFA and suitable scavengers for approximately 2 hr. The volume of the filtrate was reduced and the crude peptide was precipitated after addition of diethylether. The crude peptide precipitate was washed several times with diethylether and finally dried.

### HPLC purification of the crude peptide

The crude peptide was purified by preparative reverse phase HPLC using a conventional HPLC apparatus, such as a Gilson GX-281 with 331/332 pump combination, for binary gradient application equipped with a column, such as 5 x 25 cm Gemini NX 5u C18 110A column or a Phenomenex Luna C18 250 x21 mm 100 A, and a fraction collector using a flow 20-40 ml/min with a suitable gradient of buffer A (0.1% Fomic acid, aq.) or A (0.1% TFA, aq.) and buffer B (0.1% Formic acid, 90% MeCN, aq.) or B (0.1% TFA, 90% MeCN, aq.). Fractions were analyzed by analytical HPLC and MS and selected fractions were pooled and lyophilized. The final product was characterized by HPLC and MS.

### Formation of the disulfide bond

Following purification and lyophilisation of the crude linear peptide, the peptide was redissolved in 0.1% TFA in water, acetonitrile and acetic acid. The concentration of the peptide solution was kept at approx. 1-2 mg/ml. A solution of iodine in methanol (approx. 1.5 equiv.) was added drop-wise during stirring until the peptide solution obtained an orange colour. After 10-15 min, the reaction was completed and excess iodine was quenched with a solution of ascorbic acid in water (1 equiv.) until the peptide solution turned colourless. The peptide solution was diluted with water before purification on preparative HPLC.

### Analytical HPLC

Final purities were determined by analytic HPLC (Agilent 1100/1200 series) equipped with auto sampler, degasser, 20 µl flow cell and Chromeleon software. The HPLC was operated with a flow of 1.2 ml/min at 40°C using an analytical column, such as Kinetex 2.6 µm XB-C18 100A 100X8,6 mm column. The compound was detected and quantified at 215 nm. Buffers A (0.1% TFA, aq.) and buffer B (0.1% TFA, 90% MeCN, aq.).

### Mass spectroscopy

Final MS analysis were determined on a conventional mass spectroscopy, e.g. Waters Xevo G2 TOF, equipped with electrospray detector with lock-mass calibration and MassLynx software. It was operated in positive mode using direct injection and a cone voltage of 15V (1 TOF), 30 V (2 TOF) or 45 V (3 TOF) as specified on the chomatogram. Precision was 5 ppm with a typical resolution of 15,000-20,000.

### Synthesis of compound No 24:

Ac-IC(1)IWQDWGEHRC(1)TEGE-NH₂

Solid phase peptide synthesis was performed on a Symphony X Synthesizer using standard Fmoc chemistry. TentaGel S RAM (2.51 g; 0.23 mmol/g) was swelled in DMF (20 ml) and the Fmoc-group was deprotected according to the procedure described above.

### Amino acid coupling

Suitable protected Fmoc-amino acids according to the sequence were coupled as described above using HATU as coupling reagent. All amino acid couplings were performed at rt.

### Fmoc deprotection

Fmoc deprotection was performed according to the procedure described above.

### Cleavage of the peptide from the solid support

The peptide-resin was washed with EtOH (3 x 10 ml) and Et2O (3 x 10 ml) and dried to constant weight at room temperature (rt). The peptide was released from the resin by treatment with TFA/DODT (95/5; 60 ml, 2 h; rt).The volume of the filtrate was reduced and the crude peptide was precipitated after addition of diethylether. The crude peptide precipitate was washed several times with diethylether and eventually dried to yield 760 mg crude peptide product (purity ~30%).

### HPLC purification of the crude linear peptide

The crude peptide was purified by preparative reverse phase HPLC using a Gilson GX-281with 331/332 pump combination for binary gradient application equipped with a 5 x *25 cm Gemini NX 5u C18* 110A, column and a fraction collector and run at 35 ml/min with a gradient of buffer A (0.1% TFA, aq.) and buffer B (0.1% TFA, 90% MeCN, aq.) gradient from 20%B to 45%B in 47 min. Fractions were analyzed by analytical HPLC and MS and relevant fractions were pooled and lyophilized to yield 190 mg, with a purity of 85% as characterized by HPLC and MS as described above. Calculated monoisotopic MW 2001.58 m/z, Found 2001.81 m/z.

### Formation of the disulfide bond

The 190 mg purified linear peptide was dissolved in 220 ml water/acetonitrile (65% / 35%) with 0.1% TFA. A solution of iodine in methanol (2.2 mL, approx. 1.5 equiv. iodine) was added drop-wise during stirring until the peptide solution obtain an orange colour. The reaction was followed by analytic HPLC and the reaction were found to be complete after 10-15 min. Excess iodine was reduced with a solution of ascorbic acid in water (220 µL, approx.1 equiv.) until the peptide solution turned colourless. The volume of the peptide solution was reduced slightly through rotary evaporation before purification on preparative HPLC.

### HPLC purification of the oxidized peptide

The crude peptide was purified by preparative reverse phase HPLC using a Gilson GX-281with 331/332 pump combination for binary gradient application equipped with a 5 x *25 cm Gemini NX 5u C18* 110A, column and a fraction collector and run at 35 ml/min with a gradient of buffer A (0.1% TFA, aq.) and buffer B (0.1% TFA, 90% MeCN, aq.) gradient from 20%B to 45%B in 47 min. Fractions were analyzed by analytical HPLC and MS and relevant fractions were pooled and lyophilized to yield 138 mg, with a purity of 92% as characterized by HPLC and MS as described above. Calculated monoisotopic MW 1999.83 m/z, Found 1999.54 m/z.

### Synthesis of compound No 119

Ac-SEFC(1)I[1-Me-Trp]QDWGEHRC(1)[Sar]EGA-K([17-carboxy- heptadecanoyl][γGlu]G[Peg3][γGlu][Peg3])-NH₂

Solid phase peptide synthesis was performed on a Symphony X Synthesizer using standard Fmoc chemistry. TentaGel S RAM (3 x ~1.3 g; 0.22 mmol/g) was swelled in DMF (3 x 10 ml) prior to use and the Fmoc-group was deprotected according to the procedure described above.

### Amino acid coupling

Suitable protected Fmoc-amino acids according to the sequence were coupled as described above using HATU as coupling reagent. All couplings were performed at rt. The lysine used for the incorporation of the branched moiety was incorporated as Fmoc-Lys(Dde)-OH for orthogonal coupling of the side chian.

### Fmoc deprotection

Fmoc deprotection was performed according to the procedure described above.

### Side chain acylation

While the peptide was still attached to the resin, the orthogonal side-chain protective group (Dde) was selectively cleaved using freshly prepared hydrazine hydrate (4%) in NMP for 2 x 15 min. The unprotected lysine side-chain was doubled coupled with Fmoc-Peg3-OH followed by single couplings with Fmoc-Glu-OtBu, Fmoc-Peg3-OH, Fmoc-Gly-OH, Fmoc-Glu-OtBu and lastly the fatty acid moiety 17-carboxy-heptadecanoic acid mono tert-butyl ester using standard coupling conditions.

### Cleavage of the peptide from the solid support

The peptide-resin was washed with EtOH (3 x 15 ml) and Et2O (3 x 150 ml) and dried to constant weight at rt. The peptide was cleaved from the resin by treatment with TFA/DODT (95/5; 120 ml, 2 h; rt). The volume of the filtrate was reduced and the crude peptide was precipitated after addition of diethylether. The crude peptide precipitate was washed several times with diethylether and finally dried to yield 2.36 g crude peptide product (purity ~41-48%).

### HPLC purification of the crude linear peptide

The crude peptide was purified by preparative reverse phase HPLC using a Gilson GX-281 with 331/332 pump combination for binary gradient application equipped with a *5 x* 25 cm *Gemini NX 5u C18* 110A, column and a fraction collector and run at 35 ml/min with a gradient of buffer A (0.1% TFA, aq.) and buffer B (0.1% TFA, 90% MeCN, aq.) gradient from 30%B to 60%B in 47 min. Fractions were analyzed by analytical HPLC and MS and relevant fractions were pooled and lyophilized to yield 744 mg, with a purity of 84% as characterized by HPLC and MS as described above. Calculated monoisotopic MW 3207.47 m/z, Found 3207.32 m/z.

### Formation of the disulfide bond

The 744 mg purified linear peptide was dissolved in 350 ml 0.1% TFA in water, 150 ml acetonitrile and 100 ml acetic acid giving a clear solution. A solution of iodine in methanol (4.7 mL, approx. 1.5 equiv. iodine) was added drop-wise during stirring until the peptide solution obtained an orange colour. The reaction was followed by analytic HPLC. After 10-15 min, the reaction was completed and excess iodine was quenched with a solution of ascorbic acid in water (150 µL, approx.1 equiv.) giving a colourless solution. The volume of the peptide solution was reduced slightly by rotary evaporation before purification on preparative HPLC.

### HPLC purification of the oxidized peptide

The crude peptide was purified by preparative reverse phase HPLC using a Gilson GX-281 with 331/332 pump combination for binary gradient application equipped with a *5 x* 25 cm

*Gemini NX 5u C18* 110A, column and a fraction collector and run at 35 ml/min with a gradient of buffer A (0.1% TFA, aq.) and buffer B (0.1% TFA, 90% MeCN, aq.) gradient from 30%B to 60%B in 47 min. Fractions were analysed by analytical HPLC and MS and relevant fractions were pooled and lyophilized to yield 510 mg, with a purity of 91% as characterized by HPLC and MS as described above. Calculated monoisotopic MW 3205.47 m/z, Found 3205.23 m/z.

### Synthesis of compound No 146

Ac-SEFA(1)I[1-Me-Trp]QDWGEHRhC(1)[Sar]EGE[Peg3][Peg3]-K([17-carboxy-heptadecanoyl][γGlu]G[γGlu])-NH2

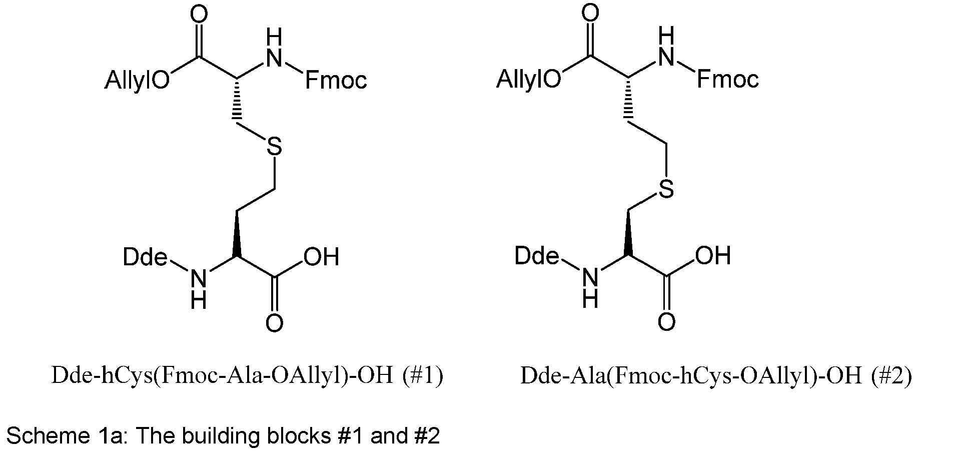

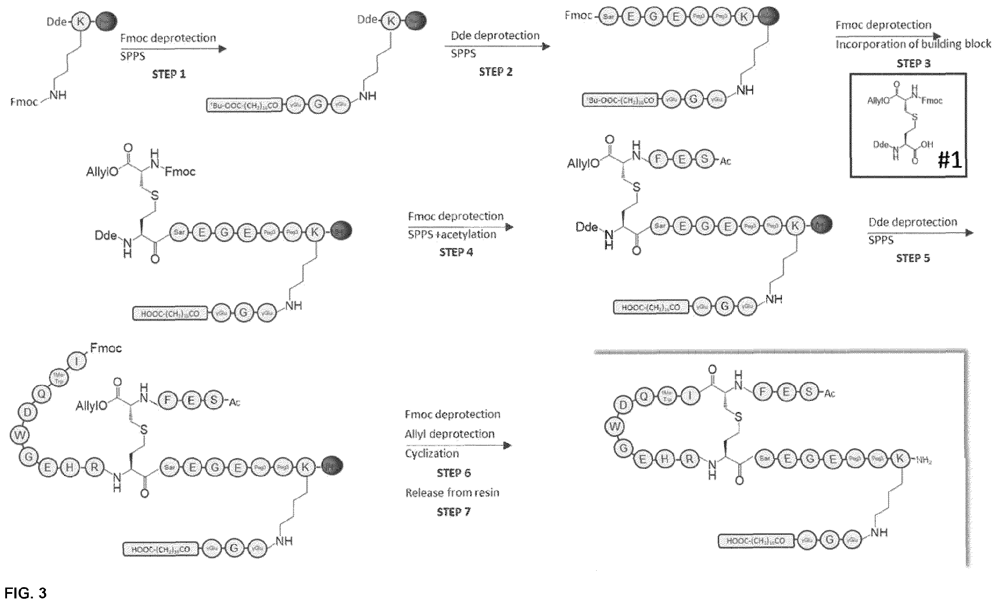

An outline of the synthetic route for compound 146 (Scheme 1b) is shown in Figure 3.

### General

Solid phase peptide synthesis was performed manual using standard Fmoc chemistry. Rink amide-MBHA (0.5 mmol, 0.214 mmol/g, ~2.3 g) was swelled in DMF:DCM overnight prior to use and the Fmoc-group was deprotected according to the procedure described above.

### Amino acid coupling

Suitable protected Fmoc-amino acids according to the sequence were coupled as described above using HBTU:NMM or HATU:NMM as coupling reagent using between 2-3 equiv. of amino acids. All couplings were performed at room temperature (rt.) for 1-3 hr unless otherwise mentioned. The lysine used for the incorporation of the branched moiety was incorporated as Dde-Lys(Fmoc)-OH for orthogonal coupling.

### Fmoc Deprotection

Fmoc deprotection was performed according to the procedure described above.

### Step 1 and 2 (Scheme 1b)

Following attachment of the Dde-Lys(Fmoc)-OH to the resin and Fmoc deprotection, the following amino acids were coupled to the lysine ε-amine: Fmoc-Glu-OtBu, Fmoc-Peg3-OH, Fmoc-Gly-OH, Fmoc-Glu-OtBu and lastly the fatty acid moiety 17-carboxy-heptadecanoic acid mono tert-butyl ester using HATU:NMM as coupling reagent. While the peptide was still attached to the resin, the protective group (Dde) was selectively cleaved using freshly prepared hydrazine hydrate (2-4%) in NMP for 2 x 15 min. Following a thorough wash with NMP the C-terminal amino acids were coupled using standard conditions (HATU:NMM in most cases) ending with coupling of the sarcosine.

### Step 3 and 4 (Scheme 1b)

Following a standard Fmoc deprotection, the building block Dde-hCys(Fmoc-Ala-OAllyl)-OH (#1, scheme 1a) was incorporated using 3 equiv. of #1 and HATU:NMM (3:6) for 2.5 hr. rt. The N-terminal amino acids was coupled using 3 equiv. of amino acids and HBTU:NMM (3:6) for 1 hour at rt. ending with acetylation of the N-terminal using Ac₂O:DCM (1:2) for 2 hr.

### Step 5 (Scheme 1b)

The synthesis was continued by removing the Dde protection group from the N-terminal amine using freshly prepared hydrazine hydrate (4%) in NMP for 2 x 15 min. Following a thorough wash with NMP the free amine and carboxylic acid were coupled forming a lactam using 3 equiv. of amino acids and HATU:NMM (3:6) for 1 hr at rt.

### Step 6 (Scheme 1b)

Following assembling of the branched peptide on-resin the allyl group was removed by treating the resin with a solution of Pd(P(C₆H₅)₃]₄ in CHCl₃/ AcOH/NMM for 3 hr. The resin was washed thoroughly and the Fmoc group was removed using standard deprotection conditions. The lactam was formed using PyAOP, HOBt and DIPEA (3:5:5) in DMF overnight.

### Cleavage of the peptide from the solid support (Step 7, scheme 1b)

The peptide-resin was washed with EtOH (3 x 15 ml) and Et₂O (3 x 150 ml) and dried. The peptide was released from the resin by treatment with TFA/EDT/Thioanisole/Phenol/H₂O (87.5/2.5/5/2.5/2.5; 35 ml, 2 hr; rt.). The volume of the filtrate was reduced and the crude peptide was precipitated after addition of diethylether. The crude peptide precipitate was washed several times with diethylether and finally dried to yield 1.4 g crude peptide product (purity 23%).

### HPLC purification of the final peptide

The crude peptide was purified by preparative reverse phase HPLC equipped with a 5 x 25 *cm Phenomenex Luna* C18 110A, column and a fraction collector and run at 35 ml/min with a gradient of buffer A (0.1% TFA, aq.) and buffer B (0.1% TFA, 90% MeCN, aq.) gradient from 30%B to 60%B in 47 min. Fractions were analyzed by analytical HPLC and MS and relevant fractions were pooled and lyophilized to yield 125.5 mg, with a purity of 90.3% as characterized by HPLC and MS as described above. Calculated monoisotopic MW 3245.52 m/z, Found 3245.32 m/z.

**Table 1a: Reference and disulfide-linked compounds:**

| **Compound** | **Sequence** |
|---|---|
| Compstatin 1-13 | H-IC(1)VVQDWGHHRC(1)T-NH₂ |
| Ac-compstatin | Ac-IC(1)VVQDWGHHRC(1)T-NH₂ |
| 4W9A* | Ac-IC(1)VWQDWGAHRC(1)T-NH₂ |
| Cp40* | H-{d}YIC(1)V[1-Me-Trp]QDW[Sar]AHRC(1)[N-Me-Ile]-NH₂ |
| A | Ac-IC(1)VWQDWGEHRC(1)T-NH₂ |
| B | Ac-IC(1)VWQDWGSHRC(1)T-NH₂ |
| C | Ac-ESSAIC(1)VWQDWGEHRC(1)T-NH₂ |
| D | Ac-IC(1)VWQDWGEHRC(1)TGAES-NH₂ |
| E | Ac-IC(1)VWQDWGAHSC(1)T-NH₂ |
| F | Ac-IC(1)VWQDWGEHSC(1)T-NH₂ |
| G | Ac-IC(1)VWQDWGEHRC(1)S-NH₂ |
| H | Ac-EGSAIC(1)VWQDWGEHRC(1)[Sar]E-NH₂ |
| J | Ac-IC(1)VWQDWGEHRC(1)TEGE-NH₂ |
| 1 | Ac-IC(1)IWQDWGAHRC(1)T-NH₂ |
| 2 | Ac-IC(1)IWQDWGEHRC(1)T-NH₂ |
| 3 | Ac-ESSAIC(1)IWQDWGEHRC(1)T-NH₂ |
| 4 | Ac-IC(N)I[1-Me-Trp]QDWGEHRC(1)T-NH₂ |
| 5 | Ac-IC(1)IWQDWGKHRC(1)T-NH₂ |
| 6 | Ac-IC(1)IWQDWGSHRC(1)T-NH₂ |
| 7 | Ac-IC(1)IWQKWGEHRC(1)T-NH₂ |
| 8 | Ac-IC(1)IWQKWGAHRC(1)TGAES-NH₂ |
| 9 | Ac-YC(1)IWQDWGEHRC(1)T-NH₂ |
| 10 | Ac-ESSAYC(1)IWQDWGEHRC(1)T-NH₂ |
| 11 | Ac-[Sar]C(1)IWQDWGEHRC(1)T-NH₂ |
| 12 | Ac-IC(1)IWQDWGAHRC(1)E-NH₂ |
| 13 | Ac-IC(1)IWQDWGEHRC(1)[Sar]-NH₂ |
| 14 | Ac-ESSAIC(1)IWQDWGEHRC(1)TGAES-NH₂ |
| 15 | Ac-IC(1)IWQDWGEHRC(1)TGAES-NH₂ |
| 16 | Ac-IC(1)IWQEWGEHRC(1)T-NH₂ |
| 17 | Ac-IC(1)IWQDWGDHRC(1)T-NH₂ |
| 18 | Ac-IC(1)IWQDWGRHRC(1)T-NH₂ |
| 19 | Ac-IC(1)IWQDWGAHSC(1)T-NH₂ |
| 20 | Ac-IC(1)IWQDWGEHSC(1)T-NH₂ |
| 21 | Ac-IC(1)IWQDWGEHRC(1)S-NH₂ |
| 22 | Ac-IC(1)IWQDWGEHRC(1)E-NH₂ |
| 23 | Ac-FC(1)IWQDWGEHRC(1)T-NH₂ |
| 24 | Ac-IC(1)IWQDWGEHRC(1)TEGE-NH₂ |
| 25 | Ac-IC(1)IWQDWGEHRC(1)TEA-NH₂ |
| 26 | Ac-IC(1)IWQDWGEHRC(1)TE-NH₂ |
| 27 | Ac-IC(1)IWQDWGEHRC(1)EGE-NH₂ |
| 28 | Ac-EGSAIC(1)IWQDWGEHRC(1)[Sar]E-NH₂ |
| 29 | Ac-EGSAIC(1)IWQDWGEHRC(1)T-NH₂ |
| 30 | Ac-EGEIC(1)IWQDWGEHRC(1)T-NH₂ |
| 31 | Ac-ESEIC(1)IWQDWGEHRC(1)T-NH₂ |
| 32 | Ac-SEIC(1)IWQDWGEHRC(1)TEA-NH₂ |
| 33 | Ac-EIC(1)IWQDWGEHRC(1)TE-NH₂ |
| 34 | Ac-EIC(1)IWQDWGEHRC(1)TEGE-NH₂ |
| 35 | Ac-EGEIC(1)IWQDWGEHRC(1)EGE-NH₂ |
| 36 | Ac-ESEIC(1)IWQDWGEHRC(1)EGE-NH₂ |
| 37 | Ac-KEKIC(1)IWQDWGEHRC(1)TEKE-NH₂ |
| 38 | Ac-EKGIC(1)IWQDWGEHRC(1)TEKP-NH₂ |
| 39 | Ac-IC(1)IWQDWGEHRC(1)TEGK-NH₂ |
| 40 | Ac-GSAIC(1)IWQDWGEHRC(1)[Sar]E-NH₂ |
| 41 | Ac-SAIC(1)IWQDWGEHRC(1)[Sar]E-NH₂ |
| 42 | Ac-SAIC(1)IWQDWGEHRC(1)TEG-NH₂ |
| 43 | Ac-FC(1)IWQDWGEHRC(1)TGAE-NH₂ |
| 44 | Ac-EGSAIC(1)IWQDWGEHRC(1)[Sar]EGE-NH₂ |
| 45 | Ac-EGSAFC(1)IWQDWGEHRC(1)[Sar]E-NH₂ |
| 46 | Ac-ESSAIC(1)IWQDWGAHRC(1)T-NH₂ |
| 47 | Ac-IC(1)IWQDWGAHRC(1)TGAES-NH₂ |
| 48 | H-{d)YIC(1)I[1-Me-Trp]QDW[Sar]AHRC(1)[N-Me-Ile]-NH₂ |
| 49 | Ac-EGSAIC(1)I[1-Me-Trp]QDWGEHRC(1)[Sar]E-NH₂ |
| 50 | Ac-EGSAIC(1)I[2-Nal]QDWGEHRC(1)[Sar]E-NH₂ |
| 51 | Ac-IC(1)I[1-Me-Trp]QDWGEHRC(1)TGAES-NH₂ |
| 52 | Ac-IC(1)I[2-Nal]QDWGEHRC(1)TGAES-NH₂ |
| 53 | Ac-EGSAFC(1)I[1-Me-Trp]QDWGEHRC(1)[Sar]E-NH₂ |
| 54 | Ac-EGSAYC(1)I[1-Me-Trp]QDWGEHRC(1)[Sar]E-NH₂ |
| 55 | Ac-EGSAIC(1)IWQDWGEHRC(1)TE-NH₂ |
| 56 | Ac-EGSAFC(1)I[1-Nal]QDWGEHRC(1)TE-NH₂ |
| 57 | Ac-EGSAFC(1)I[1-Me-Trp]QDWGEHRC(1)TE-NH₂ |
| 58 | Ac-EGSAFC(1)I[1-Me-Trp]QDWGEHRC(1)EGE-NH₂ |
| 59 | Ac-EGSAYC(1)I[1-Me-Trp]QDWGEHRC(1)TE-NH₂ |
| 60 | Ac-EGSAFC(1)I[2-Nal]QDWGEHRC(1)TE-NH₂ |
| 61 | Ac-FC(1)I[1-Me-Trp]QDWGEHRC(1)TGAES-NH₂ |
| 62 | Ac-YC(1)I[1-Me-Trp]QDWGEHRC(1)TGAES-NH₂ |
| 63 | Ac-FC(1)I[1-Nal]QDWGEHRC(1)TGAES-NH₂ |
| 64 | Ac-FC(1)I[2-Nal]QDWGEHRC(1)TGAES-NH₂ |
| 65 | Ac-YC(1)I[2-Nal]QDWGEHRC(1)TGAES-NH₂ |
| 66 | Ac-YC(1)IWQDWGEHRC(1)TGAES-NH₂ |
| 67 | Ac-SEFC(1)I[1-Me-Trp]QDWGEHRC(1)TGAES-NH₂ |
| 68 | Ac-YC(1)I[1-Me-Trp]QDWGEHRC(1)TEAGS-NH₂ |
| 69 | Ac-YC(1)I[1-Me-Trp]QDWGEHRC(1)TESGA-NH₂ |
| 70 | Ac-EGSAYC(1)I[1-Me-Trp]QEWGEHRC(1)[Sar]E-NH₂ |
| 71 | Ac-SEYC(1)I[1-Me-Trp]QDWGEHRC(1)[Sar]EA-NH₂ |
| 72 | Ac-FC(1)I[1-Me-Trp]QDW[Sar]EHRC(1)TGAES-NH₂ |
| 73 | H-{d}YFC(1)I[1-Me-Trp]QDW[Sar]EHRC(1)TGAES-NH₂ |
| 74 | Ac-SEFC(1)I[1-Me-Trp]QDWGEHRC(I)[Sar]GAES-NH₂ |
| 75 | Ac-SEFC(1)I[1-Me-Trp]QDWGEHRC(1)[Sar]EA-NH₂ |
| 76 | Ac-SEFC(1)I[1-Me-Trp]QDW[Sar]EHRC(1)[Sar]EA-NH₂ |
| 77 | Ac-SEFC(1)I[1-Me-Trp]QDW[Sar]EHRC(1)TEA-NH₂ |
| 78 | Ac-SEFC(1)I[1-Me-Trp]QDWGEHRC(1)[Sar]E-NH₂ |
| 79 | Ac-SEFC(1)I[1-Me-Trp]QDW[Sar]EHRC(1)[Sar]E-NH₂ |
| 80 | Ac-EFC(1)I[1-Me-Trp]QDWGEHRC(1)[Sar]EA-NH₂ |
| 81 | Ac-SE[Sar]C(1)I[1-Me-Trp]QDWGEHRC(1)[Sar]EA-NH₂ |
| 82 | Ac-SE[Sar]C(1)I[1-Me-Trp]QDWGEHRC(1)TEA-NH₂ |
| 83 | Ac-SEFC(1)I[1-Me-Trp]QEWGEHRC(1)[Sar]EA-NH₂ |
| 84 | Ac-SEFC(1)I[1-Me-Trp]QDWGEHRC(1)SEA-NH₂ |
| 85 | Ac-EFC(1)I[1-Me-Trp]QDWGEHRC(1)ES-NH₂ |
| 86 | Ac-SEFC(1)I[1-Me-Trp]QDWGEHKC(1)[Sar]EA-NH₂ |
| 87 | Ac-GEFC(1)I[1-Me-Trp]QDWGEHRC(1)[Sar]EA-NH₂ |
| 88 | Ac-GE[Sar]C(1)I[1-Me-Trp]QDWGEHRC(1)TEA-NH₂ |
| 89 | Ac-SE[Sar]C(1)I[1-Me-Trp]QEW[Sar]EHRC(1)TEA-NH₂ |
| 90 | Ac-SE[Sar]C(1)I[1-Me-Trp]QEWGEHRC(1)[Sar]EA-NH₂ |
| 91 | H-{d}Y[Sar]C(1)I[1-Me-Trp]QDWGEHRC(1)TEA-NH₂ |
| 92 | Ac-IC(1)IWQDWGEHRC(1)TEG-K([15-carboxypentadecanoyl][γGlu])-NH₂ |
| 93 | Ac-IC(1)IWQDWGEHRC(1)TEG-K([15-carboxypentadecanoyl][γGlu][Peg3][Peg3])-NH₂ |
| 94 | Ac-IC(1)IWQDWGEHRC(1)TEGE-K([15-carboxypentadecanoyl][γGlu][Peg3][Peg3])-NH₂ |
| 95 | Ac-IC(1)IWQDWGEHRC(1)TEG-K((15-carboxypentadecanoyl)-[(Piperazine-1-yl)-acetyl][Peg3][Peg3])-NH₂ |
| 96 | Ac-IC(1)IWQDWGEHRC(1)TEG-K([17-carboxyheptadecanoyl][γGlu][Peg3][Peg3])-NH₂ |
| 97 | Ac-IC(1)IWQDWGEHRC(1)TEGE-K([17-carboxyheptadecanoyl][γGlu][Peg3][Peg3])-NH₂ |
| 98 | Ac-IC(1)IWQDWGEHRC(1)TEG-K([19-carboxynonadecanoyl][γGlu][Peg3][Peg3])-NH₂ |
| 99 | [15-Carboxy-pentadecanoyl]-ESSAIC(1)IWQDWGEHRC(1)TEGE-NH₂ |
| 100 | Ac-[K([15-carboxy-pentadecanoyl][γGlu][Peg3]-[Peg3]GSAIC(1)IWQDWGEHRC(1)TEGE-NH₂ |
| 101 | Ac-EGSAIC(1)IWQDWGEHRC(1)TEG-K([15-carboxypentadecanoyl][γGlu])-NH₂ |
| 102 | Ac-FC(1)I[1-Me-Trp]QDWGEHRC(1)TGAES-K([15-carboxy-pentadecanoyl][γGlu][Peg3][Peg3])-NH₂ |
| 103 | Ac-EGSAYC(1)I[1-Me-Trp]QDWGEH-[K([15-carboxypentadecanoyl][γGlu][Peg3][Peg3])]-C(1)[Sar]E-NH₂ |
| 104 | Ac-EGSAYC(1)I[1-Me-Trp]QDWGEHRC(1)[Sar]EG-K([15-carboxy-pentadecanoyl][γGlu][Peg3][Peg3])-NH₂ |
| 105 | Ac-SAYC(1)I[1-Me-Trp]QDWGEHRC(1)[Sar]E-K([17-carboxy-heptadecanoyl][γGlu]KG[γGlu])-NH₂ |
| 106 | Ac-SAYC(1)I[1-Me-Trp]QDWGEHRC(1)[Sar]EK([17-carboxy-heptadecanoyl][γGlu]G[γGlu])-NH₂ |
| 107 | [15-Carboxy-pentadecanoyl]-EGSEYC(1)I[1-MeTrp]QDWGEHRC(1)[Sar]E-NH₂ |
| 108 | [17-Carboxy-heptadecanoyl]-EGSEYC(1)I[1-MeTrp]QDWGEHRC(1)[Sar]E-NH₂ |
| 109 | Ac-EGSAYC(1)I[1-Me-Trp]QDWGEHRC(1)[Sar]EGE-K([17-carboxy-heptadecanoyl][γGlu]G[γGlu])-NH₂ |
| 110 | Ac-EGSAYC(1)I[1-Me-Trp]QDWGEHRC(1)[Sar]EGK-K([17-carboxy-heptadecanoyl][γGlu]G[γGlu])-NH₂ |
| 111 | Ac-EGSAYC(1)I[1-Me-Trp]QDWGEHRC(1)[Sar]EK[γGlu]-K([17-carboxy-heptadecanoyl][γGlu][Peg3][Peg3])]-NH₂ |
| 112 | Ac-SEYC(1)I[1-Me-Trp]QDWGEHRC(1)[Sar]EGA-K([17-carboxy-heptadecanoyl][γGlu]G[γGlu])-NH₂ |
| 113 | Ac-ASGEYC(1)I[1-Me-Trp]QDWGEHRC(1)[Sar]EGE-K([17-carboxy-heptadecanoyl][γGlu]G[γGlu])-NH₂ |
| 114 | Ac-SEYC(1)I[1-Me-Trp]QDWGEHRC(1)[Sar]EGE-K([17-carboxy-heptadecanoyl][γGlu]G[γGlu])-NH₂ |
| 115 | Ac-SEYC(1)I[1-Me-Trp]QDWGEHRC(1)[Sar]EGK-K[17-carboxy-heptadecanoyl][γGlu]G[γGlu])]-NH₂ |
| 116 | Ac-SEYC(1)I[1-Me-Trp]QDWGEHRC(1)[Sar]EGE-K([17-carboxy-heptadecanoyl][γGlu]K[γGlu])-NH₂ |
| 117 | Ac-SEYC(1)I[1-MeTrp]QDWGEHRC(1)[Sar]EGE[Peg3][Peg3]-K([17-carboxy-heptadecanoyl][γGlu]G[γGlu])-NH₂ |
| 118 | Ac-SEYC(1)I[1-Me-Trp]QDWGEHRC(1)[Sar]EGA-K([17-carboxy-heptadecanoyl][γGlu]G[Peg3][γGlu][Peg3])-NH₂ |
| 119 | Ac-SEFC(1)I[1-Me-Trp]QDWGEHRC(1)[Sar]EGA-K([17-carboxy-heptadecanoyl][γGlu]G[Peg3][γGlu][Peg3])-NH₂ |
| 120 | Ac-SEFC(1)I[1-Me-Trp]QEWGEHRC(1)[Sar]EGA-K([17-carboxy-heptadecanoyl][γGlu]G[Peg3][γGlu][Peg3])-NH₂ |
| 121 | Ac-SEYC(1)I[1-Me-Trp]QEW[Sar]EHRC(1)[Sar]EK[γGlu]A-K([17-carboxy-heptadecanoyl][γGlu]G[Peg3][γGlu][Peg3])-NH₂ |
| 122 | Ac-SEYC(1)I[1-Me-Trp]QEWGEHRC(1)[Sar]EGA-K([17-carboxy-heptadecanoyl][γGlu]G[Peg3][γGlu][Peg3])-NH₂ |
| 123 | Ac-SEFC(1)I[1-Me-Trp]QDWGEHRC(1)[Sar]EGE-[Peg3][Peg3]-K([17-carboxy-heptadecanoyl][γGlu]G[γGlu])-NH₂ |
| 124 | Ac-SEFC(1)I[1-Me-Trp]QEWGEHRC(1)[Sar]EGE-[Peg3][Peg3]-K([17-carboxy-heptadecanoyl][γGlu]-G[γGlu])]-NH₂ |
| 125 | Ac-SEYC(1)I[1-MeTrp]QEWGEHRC(1)[Sar]EGE[Peg3][Peg3]-K([17-carboxyheptadecanoyl][γGlu]G[γGlu])-NH₂ |
| 126 | Ac-SEFC(1)I[1-Me-Trp]QDWGEHRC(1)TEGE[Peg3][Peg3]-K([17-carboxy-heptadecanoyl][γGlu]G[γGlu])-NH2 |
| 127 | Ac-SEFC(1)I[1-Me-Trp]QDWGEHRC(1)-[Sar]EGE[Peg3][Peg3]-K([15-carboxypentadecanoyl][γGlu]-G[γGlu])-NH₂ |
| 128 | Ac-SEFC(1)I[1-Me-Trp]QDWGEHRC(1)[Sar]EGE-[Peg3][Peg3]-K([19-carboxy-nonadecanoyl][γGlu]G[γGlu])-NH₂ |
| 129 | Ac-SEFC(1)I[1-Me-Trp]QDWGEHRC(1)[Sar]EGEGGG-K([17-carboxy-heptadecanoyl][γGlu]G[γGlu])-NH₂ |
| 130 | Ac-SEFC(1)I[1-Me-Trp]QDWGEHRC(1)TEGEGGG-K([17-carboxy-heptadecanoyl][γGlu]G[γGlu])-NH₂ |
| 131 | Ac-SEFC(1)I[1-Me-Trp]QDWGEHRC(1)TEGEGGG-K([15-carboxy-pentadecanoyl][γGlu]G[γGlu])-NH₂ |
| 132 | Ac-SEFC(1)I[1-Me-Trp]QDWGEHRC(1)[Sar]EK[γGlu]GGG-K([17-carboxy-heptadecanoyl][γGlu]G[γGlu])-NH₂ |
| 133 | Ac-SEFC(1)I[1-Me-Trp]QDWGEHRC(1)TEK[γGlu]GGG-K([17-carboxy-heptadecanoyl][γGlu]G[γGlu])-NH₂ |
| 134 | Ac-EFC(1)I[1-Me-Trp]QDWGEHRC(1)EGE-K([17-carboxyheptadecanoyl][γGlu]G[γGlu])-NH₂ |
| 135 | Ac-SEFC(1)I[1-Me-Trp]QDWGEHRC(1)TGAES-K([15-carboxy-hexadecanoyl][γGlu]G[γGlu])-NH₂ |
| 136 | Ac-SEFC(1)I[1-Me-Trp]-QDWGEHRC(1)TEGE-[8-aminooctanoyl]-K([17-carboxy-heptadecanoyl]-[γGlu]G[γGlu])-NH₂ |
| 137 | Ac-SEFC(1)I[1-Me-Trp]QDWGEHRC(1)TEGE-[8-aminooctanoyl]-E-K([17-carboxy-heptadecanoyl]-[γGlu]G[γGlu])-NH₂ |
| 138 | Ac-SEFC(1)I[1-Me-Trp]QDWGEHRC(1)[Sar]EGE-[Peg3]-K([17-carboxy-heptadecanoyl][γGlu]G[γGlu])-NH₂ |
| 139 | Ac-SEFC(1)I[1-Me-Trp]QDWGEHRC(1)[Sar]EGESES-K([17-carboxy-heptadecanoyl][γGlu]G[γGlu]])-NH₂ |
| 140 | Ac-SEFC(1)I[1-Me-Trp]QDWGEHRC(1)[Sar]EGE[Peg3]ES-K([17-carboxy-heptadecanoyl][γGlu]G[γGlu])-NH₂ |
| 141 | Ac-SEFC(1)I[1-Me-Trp]QDWGEHRC(1)[Sar]EGESES-K([17-carboxy-heptadecanoyl][γGlu])-NH₂ |
| 142 | Ac-SEFC(1)I[1-Me-Trp]QDWGEHRC(1)TEGE[Peg3]ES-K([17-carboxy-heptadecanoyl][γGlu])-NH₂ |
| 143 | Ac-SEFC(1)I[1-MeTrp]QDWGEHR[C(1)[Sar]EGE[Peg3][Peg3][Peg3]-K([17-carboxy-heptadecanoyl][γGlu]G[γGlu])-NH₂ |
| 144 | Ac-SEFC(1)I[1-MeTrp]QDW[Sar]EHRC(1)[Sar]E[Peg3][Peg3]-K([17-carboxyheptadecanoyl][γGlu]G[γGlu])-NH₂ |
| 145 | Ac-EF[C(1)I[1-Me-Trp]QDWGEHRC(1)[Sar]EA-[Peg3][Peg3]-K([17-carboxy-heptadecanoyl][γGlu]G[γGlu])-NH₂ |

| | |
|---|---|
| *: 4W9A - described by Mallik et al., J. Med. Chem. 2005, 48, 274-286 ("V4W/H9A"). Cp40- decribed by Qu et al., Immunobiology 2013, 281(4): 496-505 (also referred to in that paper as "peptide 14). | |

**Table 1b Delta-cystathionine (Ctt2) compounds**

| **Compound** | **Sequence** |
|---|---|
| 146 | Ac-SEFA(1)I[1-Me-Trp]QDWGEHRhC(1)[Sar]EGE[Peg3][Peg3]-K([17-carboxy-heptadecanoyl][γGlu]G[γGlu])-NH₂ |
| 147 | Ac-SEFA(1)I[1-Me-Trp]QDW[Sar]EHRhC(1)[Sar]E[Peg3][Peg3]-K([17-carboxy-heptadecanoyl][γGlu]G[γGlu])-NH₂ |
| 148 | Ac-SEFA(1)I[1-Me-Trp]QDWGEHRhC(1)TEGE[Peg3]ES-K([17-carboxy-heptadecanoyl][γGlu])-NH₂ |
| 149 | Ac-GEFA(1)I[1-Me-Trp]QDW[Sar]EHRhC(1)[Sar]EAE[Peg3][Peg3]-K([17-carboxy-heptadecanoyl][γGlu]G[γGlu])-NH₂ |
| 150 | Ac-SEFA(1)I[1-Me-Trp]QDW[Sar]EHRhC(1)[Sar]EGE[Peg3]ES-K([17-carboxy-heptadecanoyl][γGlu]G[γGlu])-NH₂ |
| 151 | Ac-SEFA(1)I[1-Me-Trp]QDWGEHRhC(1)TGAES-NH₂ |
| 153 | Ac-SEFA(1)]I[1-Me-Trp]QEWGEHRhC(1)[Sar]EGE[Peg3][Peg3]-K([17-carboxy-heptadecanoyl][γGlu]G[γGlu])-NH₂ |
| 154 | Ac-SEFA(1)I[1-Me-Trp]QDWGEHRhC(1)[Sar]EGA-K([17-carboxyheptadecanoyl][γGlu]G[Peg3][γGlu][Peg3])-NH₂ |
| 155 | Ac-GEFA(1)I[1-Me-Trp]QEWGEHRhC(1)[Sar]EGE[Peg3]ES-K([17-carboxy-heptadecanoyl][γGlu]G[γGlu])-NH₂ |
| 156 | Ac-SEFA(1)I[1-Me-Trp]QDWGEHRhC(1)TEGE[Peg3][Peg3]-K([17-carboxy-heptadecanoyl][γGlu]G[γGlu])-NH₂ |
| 157 | Ac-SEFA(1)I[1-Me-Trp]QDWGEHRhC(1)TEGEGGG-K([17-carboxyheptadecanoyl][γGlu]G[γGlu])-NH₂ |
| 158 | Ac-EFA(1)I[1-Me-Trp]QEWGEHRhC(1)[Sar]EA[Peg3][Peg3]-K([17-carboxy-heptadecanoyl][γGlu]G[γGlu])-NH₂ |
| 159 | Ac-EGSAYA(1)I[1-Me-Trp]QDWGEHRhC(1)[Sar]EK[γGlu]-K([17-carboxy-heptadecanoyl][γGlu][Peg3][Peg3])-NH₂ |
| 160 | Ac-SEFA(1)I[1-Me-Trp]QDWGEHRhC(1)[Sar]EGE[Peg3][Peg3]-K([15-carboxy-pentadecanoyl][γGlu]G[γGlu])-NH₂ |
| 161 | Ac-EFA(1)I[1-Me-Trp]QDWGEHRhC(1)EGE-K([17-carboxyheptadecanoyl][γGlu]G[γGlu])-NH₂ |
| 162 | Ac-SEFA(1)I[1-Me-Trp]QDW[Sar]EHRhC(1)[Sar]E[Peg3][Peg3]-K([17-carboxy-heptadecanoyl][γGlu]G[γGlu])-OH |
| 163 | Ac-SEFA(1)I[1-Me-Trp]QDWGEHRhC(1)TEGE[Peg3]ES-K([17-carboxy-heptadecanoyl][γGlu])-OH |

**Table 1c Gamma-cystathionine (Ctt1) compounds**

| **Compound** | **Sequence** |
|---|---|
| 152 | Ac-SEFhC(1)I[1-Me-Trp]QDWGEHRA(1)[Sar]EGE[Peg3][Peg3]-K([17-carboxy-heptadecanoyl][γGlu]G[γGlu]-NH₂ |
| 164 | Ac-SEFhC(1)I[1-Me-Trp]QDW[Sar]EHRA(1)[Sar]E[Peg3][Peg3]-K([17-carboxy-heptadecanoyl][γGlu]G[γGlu])-NH₂ |
| 165 | Ac-SEFhC(1)I[1-Me-Trp]QDWGEHRA(1)TEGE[Peg3]ES-K([17-carboxy-heptadecanoyl][γGlu])-NH₂ |
| 166 | Ac-SEFhC(1)I[1-Me-Trp]QDW[Sar]EHRA(1)[Sar]EGE[Peg3]ES-K([17-carboxy-heptadecanoyl][γGlu]G[γGlu])-NH₂ |
| 167 | Ac-SEFhC(1)I[1-Me-Trp]QEWGEHRA(1)[Sar]EGE[Peg3][Peg3]-K([17-carboxy-heptadecanoyl][γGlu]G[γGlu]-NH₂ |

### Example 2: In vitro haemolysis assay

### Method

The *in vitro* effect of test compounds was assessed by measuring their inhibitory effect of the classical complement pathway in a haemolysis assay.

Briefly, test compounds and reference compounds were dissolved in DMSO and diluted in Tris/Casein Assay Buffer (10 mM Tris, 145 mM NaCl, 0.5 mM MgCl₂, 0.15 mM CaCl₂, and 0.1 % W/V Casein, adjusted to pH 7.4) as 9-point serial dilutions in a 96 well plate. Sensitized sheep red blood cells (RBC) coated with rabbit anti-sheep erythrocyte antiserum (Complement Technology, Inc., TX, USA) were washed in Tris/Casein Assay Buffer. 50 µL from each well of diluted compound was added to a 96-well plate containing 50 µL diluted human serum (Complement Technology, Inc., TX, USA) and incubated for 15 minutes at room temperature. The serum dilution factor was optimized for every serum batch to obtain 70-90% of maximal haemolysis using the protocol. Then 50 µL sensitized sheep red blood cells were added to all wells (10⁷ per well).

After 30 minutes of incubation at 37 °C with gentle agitation, the reaction was stopped by addition of 50 µL Tris STOP Buffer per well (10 mM EDTA, 10 mM Tris, 145 mM NaCl adjusted to pH 7.4). The RBCs were then removed by centrifugation and the resulting supernatant measured for hemolysis by absorbance at 405 nm.

The response was normalized relative to a positive and negative control (vehicle) to calculate the IC50 from the concentration response curve using the 4-parameter logistic (4PL) nonlinear model for curve fitting. All values are based on n=>2 independent determinations.

**Table 2: Effect of exchange from valine to isoleucine. Compound 1 differs from the prior art compound 4W9A only by the presence of Ile instead of Val at position 3.**

| **Comp no** | **CP hemolysis IC50 (nM)** | | **1** | **2** | **3** | **4** | **5** | **6** | **7** | **8** | **9** | **10** | **11** | **12** | **13** | |
|---|---|---|---|---|---|---|---|---|---|---|---|---|---|---|---|---|
| 1 | 150 | Ac | I | C(1) | **I** | W | Q | D | W | G | A | H | R | C(1) | T | NH₂ |
| 4W9A | 250 | | | | **V** | | | | | | | | | | | |

Further compounds were tested as shown below.

**Table 3: in vitro analysis of inhibition of hemolysis**

| **Compound** | **IC50[nM]** |
|---|---|
| Compstatin | >5µM |
| Ac-compstatin | >5µM |
| 4W9A | <500 |
| Cp40 | <100 |
| 1 | <250 |
| 2 | <100 |
| 3 | <100 |
| 4 | <100 |
| 5 | <250 |
| 6 | <250 |
| 7 | <1000 |
| 8 | <500 |
| 9 | <100 |
| 10 | <100 |
| 11 | <100 |
| 12 | <100 |
| 13 | <100 |
| 14 | <100 |
| 15 | <100 |
| 16 | <100 |
| 17 | <100 |
| 18 | <100 |
| 19 | <250 |
| 20 | <100 |
| 21 | <100 |
| 22 | <100 |
| 23 | <100 |
| 24 | <100 |
| 25 | <100 |
| 26 | <100 |
| 27 | <100 |
| 28 | <100 |
| 29 | <100 |
| 30 | <100 |
| 31 | <100 |
| 32 | <100 |
| 33 | <100 |
| 34 | <250 |
| 35 | <500 |
| 36 | <250 |
| 37 | <250 |
| 38 | <250 |
| 39 | <100 |
| 40 | <250 |
| 41 | <250 |
| 42 | <250 |
| 43 | <100 |
| 44 | <250 |
| 45 | <100 |
| 46 | <100 |
| 47 | <100 |
| 48 | <100 |
| 49 | <100 |
| 50 | <100 |
| 51 | <100 |
| 52 | <100 |
| 53 | <100 |
| 54 | <100 |
| 55 | <250 |
| 56 | <100 |
| 57 | <100 |
| 58 | <100 |
| 59 | <100 |
| 60 | <100 |
| 61 | <100 |
| 62 | <100 |
| 63 | <100 |
| 64 | <100 |
| 65 | <100 |
| 66 | <100 |
| 67 | <100 |
| 68 | <100 |
| 69 | <100 |
| 70 | <100 |
| 71 | <100 |
| 72 | <100 |
| 73 | <100 |
| 74 | <100 |
| 75 | <100 |
| 76 | <100 |
| 77 | <100 |
| 78 | <100 |
| 79 | <100 |
| 80 | <100 |
| 81 | <100 |
| 82 | <100 |
| 83 | <100 |
| 84 | <100 |
| 85 | <100 |
| 86 | <100 |
| 87 | <100 |
| 88 | <100 |
| 89 | <100 |
| 90 | <250 |
| 91 | <100 |
| 92 | <1000 |
| 93 | <500 |
| 94 | <500 |
| 95 | <500 |
| 96 | <1000 |
| 97 | <250 |
| 98 | <500 |
| 99 | <250 |
| 100 | <500 |
| 101 | <500 |
| 102 | <100 |
| 103 | <100 |
| 104 | <100 |
| 105 | <100 |
| 106 | <250 |
| 107 | <100 |
| 108 | <500 |
| 109 | <250 |
| 110 | <250 |
| 111 | <100 |
| 112 | <500 |
| 113 | <500 |
| 114 | <500 |
| 115 | <250 |
| 116 | <500 |
| 117 | <250 |
| 118 | <100 |
| 119 | <100 |
| 120 | <250 |
| 121 | <250 |
| 122 | <500 |
| 123 | <100 |
| 124 | <100 |
| 125 | <500 |
| 126 | <100 |
| 127 | <100 |
| 128 | <100 |
| 129 | <100 |
| 130 | <100 |
| 131 | <100 |
| 132 | <100 |
| 133 | <100 |
| 134 | <100 |
| 135 | <100 |
| 136 | <100 |
| 137 | <100 |
| 138 | <100 |
| 139 | <100 |
| 140 | <100 |
| 141 | <100 |
| 142 | <100 |
| 143 | <100 |
| 144 | <100 |
| 145 | <100 |
| 146 | <100 |
| 147 | <100 |
| 148 | <100 |
| 149 | <100 |
| 150 | <100 |
| 151 | <100 |
| 152 | <100 |
| 153 | <100 |
| 154 | <100 |
| 155 | <100 |
| 156 | <100 |
| 157 | <100 |
| 158 | <250 |
| 159 | <100 |
| 160 | <100 |
| 161 | <100 |
| 162 | <100 |
| 163 | <100 |
| 164 | <100 |
| 165 | <250 |
| 166 | <100 |
| 167 | <500 |

The following pairs of compounds, each of which differ only at position 3, show that the effects of replacing valine by isoleucine are seen in compounds having a variety of peptide backbone sequences.

**Table 4 : Direct comparison of valine 3 to isoleucine 3 in combination with modification at position 9, 11 and/or 13.**

| **Compound** | **CP hemolysis IC50 (nM)** | | | **1** | **2** | **3** | **4** | **5** | **6** | **7** | **8** | **9** | **10** | **11** | **12** | **13** | | |
|---|---|---|---|---|---|---|---|---|---|---|---|---|---|---|---|---|---|---|
| 2 | 94 | Ac | | I | C(1) | **I** | W | Q | D | W | G | **E** | H | R | C(1) | T | | NH₂ |
| A | 350 | | | | | **V** | | | | | | | | | | | | |
| 6 | 140 | Ac | | I | C(1) | **I** | W | Q | D | W | G | **S** | H | R | C(1) | T | | NH₂ |
| B | 360 | | | | | **V** | | | | | | | | | | | | |
| 3 | 69 | Ac | ESSA | I | C(1) | **I** | W | Q | D | W | G | **E** | H | R | C(1) | T | | NH₂ |
| C | 300 | | | | | **V** | | | | | | | | | | | | |
| 15 | 47 | Ac | | I | C(1) | **I** | W | Q | D | W | G | **E** | H | R | C(1) | T | GAES | NH₂ |
| D | 210 | | | | | **V** | | | | | | | | | | | | |
| 19 | 140 | Ac | | I | C(1) | **I** | W | Q | D | W | G | **A** | H | **S** | C(1) | T | | NH₂ |
| E | >1000 | | | | | **V** | | | | | | | | | | | | |
| 20 | 59 | Ac | | I | C(1) | **I** | W | Q | D | W | G | E | H | **S** | C(1) | T | | NH₂ |
| F | 540 | | | | | **V** | | | | | | | | | | | | |
| 21 | 77 | Ac | | I | C(1) | **I** | W | Q | D | W | G | E | H | R | C(1) | **S** | | NH₂ |
| G | 180 | | | | | **V** | | | | | | | | | | | | |
| 28 | 88 | Ac | EGSA | I | C(1) | **I** | W | Q | D | W | G | **E** | H | R | C(1) | **Sar** | E | NH₂ |
| H | 330 | | | | | **V** | | | | | | | | | | | | |
| 24 | 90 | Ac | | I | C(1) | **I** | W | Q | D | W | G | **E** | H | R | C(1) | T | EGE | NH₂ |
| J | 240 | | | | | **V** | | | | | | | | | | | | |

Isoleucine at position 3 was also demonstrated to be superior compared to other residues often considered to be "conservative" replacements for isoleucine.

**Table 5: Effect on hemolysis of different residues at position 3 Ac-IC(1)XWQDWGEHRC(1)T-NH₂**

| Compound | Position 3 (X) | IC50, CP hemolysis (nM) |
|---|---|---|
| A | Valine | 350 |
| 2 | Isoleucine | <100 |
| - | Leucine | 500 |
| - | Norvaline | >1000 |
| - | Norleucine | 480 |
| - | Phenylalanine | >10000 |
| - | Beta-Homo-Isoleucine | >10000 |

Due to the high concentration of C3 found in serum, it may be difficult to use the hemolysis assay to differentiate between compounds having very high affinity for C3.

In such circumstances, it may be possible to determine a more accurate hierarchy of binding affinity to C3 by SPR measurements using immobilized C3, as described below.

### Example 3: Solubility test

### Materials and method

### Compound solubility at 10 mg/mL

The solubility of compounds was assessed by measuring light scattering over a pH interval from pH 4 to pH 7.5.

Compounds were dissolved in a stock solution of 20 mg/mL in H₂O at pH 2.5 or pH 10. These stock solutions were diluted 1:1 with 200 mM buffered solution to reach a final solution of 10 mg/mL compound in 100 mM buffer. The 5 investigated conditions were (1) acetate pH 4.0, (2) acetate pH 5.0, (3) phosphate pH 6.0, (4) phosphate pH 7 and (5) phosphate pH 7.5.

These samples were equilibrated for 15 minutes at ambient temperature, before evaluating solubility by visual inspection and absorbance measurements in a SpectraMax 190 microplate reader (Molecular Devices).

### Visual inspection

Visual inspection included manually checking the 96 well plate for wells that are clear or non-clear. In addition to this a picture of the 96 well plate is taken.

### Microplate reader and light scattering

Absorbance was measured at four wavelengths: 280 nm, 325 nm, 340 nm and 360 nm in an UV transparent 96 well microplate in a SpectraMax 190 microplate reader (Molecular Devices). The compounds do not absorb at 325-360 nm and signal at these wavelengths are therefore an expression of light scattering, which reflects the presence of visible or sub-visible particles that are detected as increased signal.

The light scattering was normalized to the signal from pure buffer solutions (100 mM) and compound solubility was evaluated as good (+) or poor (-). The criteria for this was a combination of visual inspection and light scattering not exceeding 0.1 AU, where values below 0.1 AU are good in visually clear samples.

### Solubility of Comp No 24:

### Stock solution

Comp No 24 was carefully weighed out and dissolved in pH 2.5 H₂O-Cl. The stock solution was equilibrated 15 minutes at ambient temperature, at which point no visible particles were present. 200 mM buffer stock solutions were prepared for each pH condition.

### Solubility assay:

The formulations for solubility testing were made by mixing 50 µL Comp No 24 stock solution and 50 µL buffer stock solution with gentle mixing by pipetting the solution a couple of times. This was done for each buffer/pH condition in a UV transparent 96 well microplate (Corning 96 well REF 3635). Reference samples without Comp No 24 were made by mixing 50 µL pH 2.5 H₂O-Cl and 50 µL buffer stock solution. The plate was covered with a lid and left 15 minutes at ambient temperature before assessing solubility.

### Measuring solubility:

Solubility was assessed by visual inspection of each formulation and a picture taken in a photo box. Light scattering was measured at 280 nm, 325 nm, 340 nm and 360 nm in a SpectraMax 190 microplate reader (Molecular Devices).

The visual inspection revealed that condition 1, 2 and 3 were cloudy and condition 2 additionally contained visible precipitates. The absorbance measurement confirmed the visual evaluation with condition 1, 2 and 3 all exceeding 0.1 AU threshold. Condition 4 and 5 were thus deemed good conditions for solubility of 10 mg/mL Comp No 24.

Similarly, additional compounds were tested for solubility (Table 6).

**Table 6: Table of most soluble compounds, as tested at 10 mg/mL. "+" denotes solubility at the given condition, as determined by UV absorbance being less than 0.1 AU at 340 nm and the sample being clear when manually inspected. "-" denotes lack of solubility at the given condition, as UV absorbance at 340 nm exceeds 0.1 AU and/or it is visibly turbid or contains particles.**

| Comp No | Buffer & pH | | | | |
|---|---|---|---|---|---|
| | Condition 1 | Condition 2 | Condition 3 | Condition 4 | Condition 5 |
| | Acetate pH 4 | Acetate pH 5 | Phosphate pH 6 | Phosphate pH 7 | Phosphate pH 7.5 |
| 1 | + | - | - | - | - |
| 3 | + | - | - | + | + |
| 14 | + | - | - | + | + |
| 15 | + | - | - | + | + |
| 22 | + | - | - | + | + |
| 24 | - | - | - | + | + |
| 25 | + | - | - | + | + |
| 27 | - | - | - | + | + |
| 28 | + | + | + | + | + |
| 30 | - | - | - | + | + |
| 31 | + | + | + | + | + |
| 32 | - | - | - | + | + |
| 33 | - | - | + | + | + |
| 36 | - | - | - | + | + |
| 40 | - | - | + | + | + |
| 41 | - | - | + | + | + |
| 44 | - | - | + | + | + |
| 45 | - | + | + | + | + |
| 49 | - | - | + | + | + |
| 50 | - | - | + | + | + |
| 51 | - | - | + | + | + |
| 52 | - | - | + | + | + |
| 53 | - | - | + | + | + |
| 54 | - | - | + | + | + |
| 55 | - | - | + | + | + |
| 56 | - | - | + | + | + |
| 57 | - | - | + | + | + |
| 60 | - | - | + | + | + |
| 61 | - | - | + | + | + |
| 62 | - | - | + | + | + |
| 63 | - | - | + | + | + |
| 65 | - | - | + | + | + |
| 66 | - | - | + | + | + |
| 67 | - | - | + | + | + |
| 68 | - | - | + | + | + |
| 72 | - | - | + | + | + |
| 73 | + | - | - | + | + |
| 74 | - | + | + | + | + |
| 76 | - | - | + | + | + |
| 77 | - | - | + | + | + |
| 78 | - | - | + | + | + |
| 79 | - | - | + | + | + |
| 80 | - | - | + | + | + |
| 81 | - | - | + | + | + |

**Table 6 (contd.)**

| Comp No | Buffer & pH | | | | |
|---|---|---|---|---|---|
| | Condition 1 | Condition 2 | Condition 3 | Condition 4 | Condition 5 |
| | Acetate pH 4 | Acetate pH 5 | Phosphate pH 6 | Phosphate pH 7 | Phosphate pH 7.5 |
| 102 | - | + | + | + | + |
| 103 | - | + | + | + | + |
| 104 | - | + | + | + | + |
| 105 | - | - | + | + | + |
| 107 | - | - | + | + | + |
| 108 | - | - | + | + | + |
| 109 | - | + | + | + | + |
| 111 | - | - | + | + | + |
| 114 | + | + | + | + | + |
| 115 | - | - | + | + | + |
| 116 | - | - | + | + | + |
| 118 | - | + | + | + | + |

### Example 4: Affinity measurements by surface plasmon resonance (SPR)

### Method

Surface plasmon resonance (SPR) was used to characterize peptides with respect to their binding affinity (Kd) for C3. Human C3 (Complement tech cat #A113c) was immobilised on individual flow cells of CM5 sensor chips (GE Healthcare) using standard amine coupling to a density of approximately 3000 resonance units (RU) in a buffer consisting of 10 mM phosphate pH 7.4, 150 mM NaCl, 0.05% Tween20.

For interaction experiments a multi-cycle experiment approach was used and performed using either a Biacore^{™}T200 or Biacore^{™}X100 instrument (GE Healthcare) at 25°C. Peptides were injected in increasing concentration series (5-8 different concentrations) for 60-120 s at a flow rate of 30 µL/min in a buffer consisting of 10 mM Tris buffer at pH 7.4, with 150 mM NaCl and 0.05% Tween20. This was followed by a dissociation period for up to 10 min. The C3 surface was regenerated between runs by a 45 s injection of 3 M MgCl₂.

Sensorgrams were double-referenced (reference surface, blanks) prior to analysis of the kinetic profiles by globally fitting data to a 1:1 Langmuir binding model to obtain association and dissociation rates for calculation of the equilibrium dissociation constant Kd. Each peptide was tested at in at least 2 independent experiments.

**Table 7: Compstatin analogues binding affinities for C3 as determined by a surface plasmon resonance assay with immobilized C3.**

| **Comp. no.** | **Kd [nM]** | **N** |
|---|---|---|
| 2 | 16 | 3 |
| 4 | 1.5 | 3 |
| 15 | 14 | 3 |
| 20 | 37 | 3 |
| 21 | 16 | 3 |
| 23 | 2.8 | 3 |
| 24 | 28 | 5 |
| 28 | 44 | 3 |
| 29 | 21 | 3 |
| 43 | 3.3 | 3 |
| 48 | 0.12 | 3 |
| 49 | 3.2 | 3 |
| 50 | 13 | 3 |
| 53 | 1.4 | 3 |
| 54 | 3.0 | 3 |
| 61 | 0.33 | 3 |
| 63 | 4.3 | 3 |
| 67 | 0.68 | 7 |
| 73 | 0.30 | 3 |
| 75 | 1.5 | 3 |
| 81 | 9.7 | 3 |
| 82 | 5.4 | 3 |
| 85 | 1.3 | 3 |
| 86 | 2.6 | 3 |
| 102 | 1.7 | 3 |
| 104 | 34 | 2 |
| 106 | 5.4 | 5 |
| 107 | 6.1 | 5 |
| 111 | 8.2 | 5 |
| 117 | 24 | 3 |
| 118 | 11 | 5 |
| 119 | 9.8 | 3 |
| 120 | 28 | 3 |
| 121 | 30 | 3 |
| 122 | 63 | 3 |
| 123 | 11 | 3 |
| 124 | 31 | 3 |
| 125 | 71 | 3 |
| 126 | 5.2 | 3 |
| 127 | 8.5 | 3 |
| 128 | 6.5 | 3 |
| 130 | 4.4 | 3 |
| 139 | 7.4 | 3 |
| 140 | 7.6 | 3 |
| 141 | 6.6 | 3 |
| 142 | 4.8 | 3 |
| 144 | 1.5 | 2 |
| 146 | 0.43 | 2 |
| 147 | 0.71 | 2 |
| 148 | 3.4 | 2 |
| 149 | 0.50 | 2 |
| 150 | 0.56 | 2 |
| 151 | 1.4 | 2 |
| 152 | 16 | 2 |
| 153 | 11.1 | 2 |
| 154 | 2.4 | 2 |
| 157 | 3.5 | 2 |
| 161 | 3.4 | 2 |
| 164 | 6.4 | 2 |
| 165 | 20.7 | 2 |
| 166 | 6.5 | 2 |
| 167 | 57.4 | 2 |

The following pairs of compounds, which differ only at position 3, show the effects of replacing valine by isoleucine in different peptide backbones.

**Table 8: Binding affinity of compstatin analogues to immobilized C3 determined by a surface plasmon resonance (SPR) assay.**

| **Comp no** | **SPR Kd (nM)** | | | **1** | **2** | **3** | **4** | **5** | **6** | **7** | **8** | **9** | **10** | **11** | **12** | **13** | | |
|---|---|---|---|---|---|---|---|---|---|---|---|---|---|---|---|---|---|---|
| 2 | 16 | Ac | | I | C(1) | **I** | W | Q | D | W | G | **E** | H | R | C(1) | T | | NH₂ |
| A | 130 | | | | | **V** | | | | | | | | | | | | |
| 15 | 14 | Ac | | I | C(1) | **I** | W | Q | D | W | G | **E** | H | R | C(1) | T | GAES | NH₂ |
| D | 230 | | | | | **V** | | | | | | | | | | | | |
| 21 | 16 | Ac | | I | C(1) | **I** | W | Q | D | W | G | **E** | H | R | C(1) | **S** | | NH₂ |
| G | 160 | | | | | **V** | | | | | | | | | | | | |
| 48 | 0.12 | H | dTyr | I | C(1) | **I** | 1MeTrp | Q | D | W | Sar | A | H | R | C(1) | NMelle | | NH₂ |
| Cp40 | 0.31 | | | | | **V** | | | | | | | | | | | | |

### Example 5: Profiling of test compounds in Non-Human Primates (NHP)

Healthy male Cynomolgus monkeys (*Macaca fascicularis*) received single subcutaneous administrations of each test substance. Compounds were formulated in 20 mM phosphate adjusted with NaOH to pH 7.5 and mannitol for isotonicity and dosed at 1840 nmol/kg. Blood was collected from a femoral vein from each animal at the following times: Pre-dose, 1, 2, 4, 8, 24, 48, 72, 96 and 120 h (10 sampling times). Blood was collected into serum separation tubes and allowed to clot at room temperature. The tubes were centrifuged and resulting serum was aliquoted and snap-frozen over dry-ice and stored at nominally -80⁰C until analysis. All NHP studies were performed in accordance with animal welfare laws and regulations, including approval of the study by a local ethical review process.

Serum isolated from non-human primates at specific time points after dosing were analyzed for alternative pathway complement activity using the Complement system Alternative Pathway WIESLABO kit from Svar Life Science (previously Euro diagnostic AB, Sweden) following the manufacturer's protocol. Briefly, serum samples or controls were diluted in buffer and incubated in microtitre strips coated with specific activators of the alternative pathway. The wells were washed and formed C5b-9 was detected using included colorimetric reagents. Absorbance at 405 nm was measured. The percent activity of the alternative complement pathway was calculated for each animal and timepoint relative to the pre-dose activity (0 hours) of the individual animal with subtraction of the negative control. This reflects the pharmacological activity of the compounds.

In a separate experiment, healthy male Cynomolgus monkeys (*Macaca fascicularis*) received a 460 nmol/kg subcutaneous administrations of test substance 14 days prior to administration of 1840 nmol/kg of the same test substance. Blood was collected from a femoral vein from each animal at the following times based on the second dosing: Pre-dose, 0.25, 0.5, 2, 4, 24, 48, 72, 96 and 120 h (10 sampling times). The analysis of the sample was performed as described for the previous samples, however with single determinations of activity.

The results from the Alternative Pathway WIESLAB^{®} kit are shown in Figure 1. Figures 1a-1g show results of experiments performed without pre-dosing. Figure 1h shows the results of the single experiment performed with pre-dosing.

In Fig 1a, the non-acylated compound 61 had a relatively short duration of action despite high affinity for C3. The same is seen for the non-acylated compounds Cp40 (figure 1b) and compound 54 (figure 1e). By contrast, the acylated compounds in Fig 1b, 1c, 1d, 1e, 1f and 1g in general possessed a longer-lasting pharmacological activity in vivo when compared to the non-acylated compounds despite lower affinity. Although acylation of peptides is generally known to increase the in vivo half-life, it was surprisingly found that the in vivo duration of the pharmacological efficacy was prolonged to this extent.

In order to assess pharmacokinetic half-life (t½), serum samples isolated from non-human primates at specific time points after dosing were analysed for total drug compound after sample preparation by solid phase extraction (SPE) and liquid chromatography mass spectrometry (LC-MS/MS) using analogue internal standard. Single measurement of serum concentrations were used for calculation of the pharmacokinetic parameters using the non-compartmental approach in Phoenix WinNonlin 6.3. Plasma terminal elimination half-life (t_{½}) was determined as In(2)/λz where λz is the magnitude of the slope of the log linear regression of the log concentration versus time profile during the terminal phase.

Pharmacokinetic (PK) data are shown in Table 9.

**Table 9: PK data in NHP:**

| **Compound** | **t_{½}** |
|---|---|
| | **hours** |
| Cp40 | 31.8 |
| 54 | 9.71 |
| 61 | 23.3 |
| 104 | 96.3 |
| 106 | 93.9 |
| 107 | 20.1 |
| 111 | 157 |
| 118 | 78.7 |
| 118 | 155 |
| 119 | 139 |
| 122 | 127 |
| 123 | 105 |
| 124 | 112 |
| 139 | 82 |
| 140 | 100 |
| 141 | 145 |
| 142 | 143 |
| 146 | 117 |
| 147 | 108 |
| 148 | 129 |
| 150 | 120 |

Determinations are approximate, as t½ determined over less than three times the expected half-life.

### Example 6: Chemical stability of cystathionine-bridged compstatin analogues

### Materials and Methods

Chemical stability of compstatin analogues with cystathionine or disulfide bridge.

Stability was assessed in three formulations at 50 mg/mL peptide, F1: 20 mM phosphate pH 5.5, F2: 20 mM phosphate pH 6.5 and F3: 20 mM phosphate pH 7.5. The formulations were prepared by direct dissolution of peptide from lyophilized portions in the respective formulations. pH was adjusted to target with 1 M NaOH and 1 M HCl.

Chemical stability was assessed under accelerated conditions (14 days, 40°C) and evaluated as change in purity by reverse phase HPLC, and change in covalent oligomer content by denaturing SEC. The formulations were placed at 40°C with analysis points at T= 0, 7 and 14 days. On each time point a small volume was extracted and diluted to 2 mg/mL and tested by reverse phase HPLC and denaturing SEC.

### Reverse phase HPLC method for purity determination

Purity was assessed by reverse phase HPLC using an Ultimate 3000 system for binary gradient application equipped with a Kinetix C18 column (cat. no. 00F-4462-Y0, 150 mm x 3 mm, 2.6 µm) run at 0.5 ml/min with a gradient of buffer A (0.3% TFA, aq.) and buffer B (0.3% TFA, 90% MeCN, aq.) gradient from 40%B to 70%B over 20 min. Detection was done using a diode array detector set to 220 nm.

### Denaturing SEC method for purity determination

Covalent oligomer formation was assessed by denaturing SEC (size exclusion chromatography) using an Ultimate 3000 system equipped with a TSKgel SuperSW 2000 column (cat. no. 818674, 300 mm x 3 mm, 4 µm) run at 0.5 ml/min with isocratic flow of buffer A (0.1% TFA, 45% MeCN, aq.), 10 min. Detection was done using a diode array detector set to 215 nm.

### Chemical stability of Comp. No. 126 (disulfide) and 156 (cystathionine):

### Sample preparation:

Compounds 126 and 156 were carefully weighed out and dissolved in formulations F1, F2 and F3 to a target concentration of 50 mg/mL. Once visually dissolved, concentration was confirmed by absorbance at 280 nm, and samples were placed in stability at 40°C.

### Chemical stability assay:

Samples for analysis were extracted on T= 0, 7 and 14 days from a 40°C climate chamber. The extracted volume was dissolved 1:25 (final concentration: 2 mg/mL) in 20 mM phosphate pH 5.5, 6.5 or 7.5 and measured by reverse phase HPLC and denaturing SEC by injection of 0.4 µL (column load 0.8 µg).

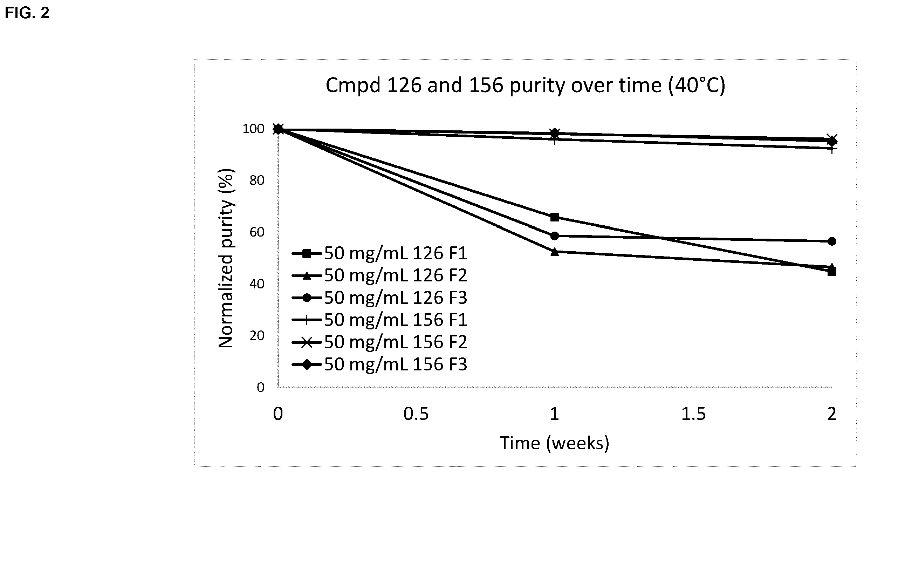

The resulting chromatograms were integrated to determine chemical purity by reverse phase HPLC and covalent oligomer content by denaturing SEC. The change in purity and corresponding increase in covalent oligomer content shows that compound 156 is significantly more stable than compound 126, as shown in Fig 2.

Chemical purity data from 2-week storage at 40°C of five disulfide based compounds (compound 111, 119, 123, 126 and 142) and five cystathionine based compounds (compounds 147, 148, 150, 153 and 156) are summarized in Table 10.

The data show that the compounds with a cystathionine bridge have significantly better chemical stability than compounds with a disulfide bridge at pH 5.5-7.5.

**Table 10: Normalized purity (HPLC) and covalent oligomer content (SEC) of peptides after 14 days storage at 40°C in formulations F1, F2 and F3.**

| | | **Purity after 14 days 40°C (%)** | | |
|---|---|---|---|---|
| **Compound number** | **Bridge chemistry** | **F1** | **F2** | **F3** |
| Compound 111 | Disulfide | 80 | 24 | 33 |
| Compound 119 | Disulfide | 23 | 31 | 46 |
| Compound 123 | Disulfide | 56 | 54 | 54 |
| Compound 126 | Disulfide | 45 | 47 | 57 |
| Compound 142 | Disulfide | 50 | 37 | 45 |
| Compound 147 | Cystathionine | 98 | 99 | 99 |
| Compound 148 | Cystathionine | 93 | 97 | 97 |
| Compound 150 | Cystathionine | 98 | 100 | 100 |
| Compound 153 | Cystathionine | 97 | 99 | 99 |
| Compound 156 | Cystathionine | 92 | 96 | 95 |

| | | **Covalent oligomer content after 14 days 40°C (%)** | | |
|---|---|---|---|---|
| **Compound number** | **Bridge chemistry** | **F1** | **F2** | **F2** |
| Compound 111 | Disulfide | 30 | 77 | 61 |
| Compound 119 | Disulfide | 80 | 66 | 48 |
| Compound 123 | Disulfide | 52 | 48 | 45 |
| Compound 126 | Disulfide | 66 | 54 | 41 |
| Compound 142 | Disulfide | 63 | 64 | 56 |
| Compound 147 | Cystathionine | 0.6 | 0.1 | 0.2 |
| Compound 148 | Cystathionine | 0.7 | 0.0 | 0.1 |
| Compound 150 | Cystathionine | 0.7 | 0.1 | 0.1 |
| Compound 153 | Cystathionine | 0.7 | 0.5 | 0.3 |
| Compound 156 | Cystathionine | 1.1 | 0.5 | 1.1 |

## Claims

1. A compstatin analogue having the sequence:
Ac-SEFA(1)I[1-Me-Trp]QDWGEHRhC(1)TEGE-[Peg3]ES-[K*]-NH₂
wherein the side chains of residues designated A(1) and hC(1) form a cystathionine bridge, and
wherein K* is a lysine residue covalently linked to a group Z¹- or Z¹-Z²-; wherein:
Z¹ is COOH-C₁₂₋₂₂alkylene-(CO)-; and
Z² is selected from:
[γGlu];
[γGlu][Peg3][Peg3];
[(Piperazine-1-yl)-acetyl][Peg3][Peg3];
[γGlu]G[γGlu];
[γGlu]K[γGlu];
[γGlu]KG[γGlu]; or
[γGlu]G[Peg3][γGlu][Peg3]
where Peg3 represents 8-amino-3,6-dioxaoctanoic acid;
or a pharmaceutically acceptable salt and/or solvate thereof.

2. A compstatin analogue or pharmaceutically acceptable salt and/or solvate thereof according to claim 1 wherein Z¹ is HOOC-(CH₂)₁₂₋₂₂-(CO)-.

3. A compstatin analogue or pharmaceutically acceptable salt and/or solvate thereof according to claim 1 or claim 2 wherein Z¹ is selected from:
HOOC-(CH₂)₁₂-(CO)-;
HOOC-(CH₂)₁₄-(CO)-;
HOOC-(CH₂)₁₆-(CO)-;
HOOC-(CH₂)₁₈-(CO)-; or
HOOC-(CH₂)₂₀-(CO)-; and
wherein Z² is selected from:
[γGlu];
[γGlu][Peg3][Peg3]-;
[(Piperazine-1-yl)-acetyl][Peg3][Peg3];
[yGlu]G[yGlu];
[yGlu]K[yGlu];
[yGlu]KG[yGlu]; or
[γGlu]G[Peg3][γGlu][Peg3].

4. A compstatin analogue or pharmaceutically acceptable salt and/or solvate thereof according to any one of claims 1 to 3 wherein Z¹- or Z¹-Z²- is selected from:
15-carboxy-pentadecanoyl;
15-carboxy-pentadecanoyl[γGlu],
15-carboxy-pentadecanoyl[γGlu][Peg3][Peg3];
19-carboxy-nonadecanoyl[γGlu][Peg3][Peg3];
15-carboxy-pentadecanoyl[(Piperazine-1-yl)-acetyl][Peg3][Peg3];
17-carboxy-heptadecanoyl[γGlu]G[γGlu];
17-carboxy-heptadecanoyl[γGlu]K[γGlu];
17-carboxy-heptadecanoyl[γGlu]KG[γGlu];
17-carboxy-heptadecanoyl[γGlu]G[Peg3][γGlu][Peg3];
15-carboxy-pentadecanoyl[γGlu]G[γGlu];
17-carboxy-heptadecanoyl;
17-carboxy-heptadecanoyl[γGlu]
19-carboxy-nonadecanoyl[γGlu]G[γGlu];and
17-carboxy-heptadecanoyl[γGlu][Peg3][Peg3].

5. A compstatin analogue according to claim 1 which is:
Ac-SEFA(1)I[1-Me-Trp]QDWGEHRhC(1)TEGE[Peg3]ES-K([17-carboxy- heptadecanoyl][γGlu])-NH₂
wherein the side chains of residues designated A(1) and hC(1) form a cystathionine bridge, or a pharmaceutically acceptable salt and/or solvate thereof.

6. A composition comprising a compstatin analogue according to any one of claims 1 to 5, or a pharmaceutically acceptable salt and/or solvate thereof, in admixture with a carrier.

7. A composition according to claim 6, wherein the composition is a pharmaceutical composition and the carrier is a pharmaceutically acceptable carrier.

8. A pharmaceutical composition comprising a compstatin analogue according to any one of claims 1 to 5, or a pharmaceutically acceptable salt and/or solvate thereof, in admixture with a pharmaceutically acceptable carrier, excipient or vehicle.

9. A compstatin analogue, or pharmaceutically acceptable salt and/or solvate thereof, according to any one of claims 1 to 5, for use in therapy.

10. A compstatin analogue, or pharmaceutically acceptable salt and/or solvate thereof, according to any one of claims 1 to 5 for use in a method of prophylaxis or treatment of age-related macular degeneration, Stargardt disease, periodontitis, diabetic retinopathy, glaucoma, uveitis, rheumatoid arthritis, spinal cord injury, stroke, multiple sclerosis, Parkinson's disease, Alzheimer's disease, cancer, and respiratory disorders such as asthma, chronic obstructive pulmonary disease (COPD), allergic inflammation, emphysema, bronchitis, bronchiecstasis, cystic fibrosis, tuberculosis, pneumonia, respiratory distress syndrome (RDS - neonatal and adult), rhinitis and sinusitis; bacterial infections such as sepsis, ischemia-reperfusion injury in various tissues, myocardial infarction, anaphylaxis, paroxysmal nocturnal hemoglobinuria, autoimmune hemolytic anemias, psoriasis, hidradentitis suppurativa, myasthenia gravis, systemic lupus erythematosus, CHAPLE syndrome, C3 glomeropathy, IgA nephropathy, atypical hemolytic uremic syndrome, Crohn's disease, ulcerative colitis or antiphospholipid syndrome.

## Patentansprüche

1. Compstatin-Analogon, das die folgende Sequenz aufweist:
Ac-SEFA(1)I[1-Me-Trp]QDWGEHRhC(1)TEGE-[Peg3]ES-[K*]-NH₂,
worin die Seitenketten der als A(1) und hC(1) bezeichneten Reste eine Cystathionin-Brücke bilden, und
worin K* ein Lysinrest ist, der kovalent an eine Gruppe Z¹- oder Z¹-Z²- gebunden ist; worin:
Z¹ COOH-C₁₂₋₂₂-Alkylen -(CO)- ist und
Z² aus Folgendem ausgewählt ist:
[γGlu],
[γGlu][Peg3][Peg3],
[(Piperazin-1-yl)-acetyl][Peg3][Peg3],
[γGlu]G[γGlu],
[γGlu]K[γGlu],
[γGlu]KG[γGlu] oder
[γGlu]G[Peg3][γGlu][Peg3],
worin Peg3 für 8-Amino-3,6-dioxaoctansäure steht;
oder ein pharmazeutisch annehmbares Salz und/oder Solvat davon.

2. Compstatin-Analogon oder ein pharmazeutisch annehmbares Salz und/oder Solvat davon nach Anspruch 1, worin Z¹ HOOC-(CH₂)₁₂₋₂₂-(CO)- ist.

3. Compstatin-Analogon oder ein pharmazeutisch annehmbares Salz und/oder Solvat davon nach Anspruch 1 oder Anspruch 2, worin Z¹ aus Folgendem ausgewählt ist:
HOOC-(CH₂)₁₂-(CO)-,
HOOC-(CH₂)₁₄-(CO)-,
HOOC-(CH₂)₁₆-(CO)-,
HOOC-(CH₂)₁₈-(CO)- oder
HOOC-(CH₂)₂₀-(CO)-; und
worin Z² aus Folgendem ausgewählt ist:
[γGlu],
[γGlu][Peg3][Peg3]-,
[(Piperazin-1-yl)-acetyl][Peg3][Peg3],
[γGlu]G[γGlu],
[γGlu]K[γGlu],
[γGlu]KG[γGlu] oder
[γGlu]G[Peg3][γGlu][Peg3].

4. Compstatin-Analogon oder ein pharmazeutisch annehmbares Salz und/oder Solvat davon nach einem der Ansprüche 1 bis 3, worin Z¹- oder Z¹-Z²- aus Folgendem ausgewählt ist:
15-Carboxypentadecanoyl,
15-Carboxypentadecanoyl[γGlu],
15-Carboxypentadecanoyl[γGlu][Peg3][Peg3],
19-Carboxynonadecanoyl[γGlu][Peg3][Peg3],
15-Carboxypentadecanoyl-[(Piperazin-1-yl)-acetyl][Peg3][Peg3]),
17-Carboxyheptadecanoyl[γGlu]G[γGlu],
17-Carboxyheptadecanoyl[γGlu]K[γGlu],
17-Carboxyheptadecanoyl[γGlu]KG[γGlu],
17-Carboxyheptadecanoyl[γGlu]G(Peg3)[γGlu]-(Peg3),
15-Carboxyhexadecanoyl[γGlu]G[γGlu],
17-Carboxyheptadecanoyl,
17-Carboxyheptadecanoyl[γGlu]],
19-Carboxynonadecanoyl[yGlu]G[yGlu] und
17-Carboxyheptadecanoyl[γGlu][Peg3][Peg3].

5. Compstatin-Analogon nach Anspruch 1, welches das folgende ist:
Ac-SEFA(1)I[1-Me-Trp]QDWGEHRhC(1)TEGE[Peg3]ES-K([17-Carboxyheptadecanoyl]- [γGlu]G[γGlu])-NH₂,
worin die Seitenketten der als A(1) und hC(1) bezeichneten Reste eine Cystathionin-Brücke bilden, oder ein pharmazeutisch annehmbares Salz und/oder Solvat davon.

6. Zusammensetzung, die ein Compstatin-Analogon nach einem der Ansprüche 1 bis 5 oder ein pharmazeutisch annehmbares Salz und/oder Solvat davon in einem Gemisch mit einem Träger umfasst.

7. Zusammensetzung nach Anspruch 6, wobei die Zusammensetzung eine pharmazeutische Zusammensetzung ist und der Träger ein pharmazeutisch annehmbarer Träger ist.

8. Pharmazeutische Zusammensetzung, die ein Compstatin-Analogon nach einem der Ansprüche 1 bis 5 oder ein pharmazeutisch annehmbares Salz und/oder Solvat davon in einem Gemisch mit einem pharmazeutisch annehmbar Träger, Hilfsstoff oder Vehikel umfasst.

9. Compstatin-Analogon oder eiin pharmazeutisch annehmbares Salz und/oder Solvat davon nach einem der Ansprüche 1 bis 5 zur Verwendung in einer Therapie.

10. Compstatin-Analogon oder ein pharmazeutisch annehmbares Salz und/oder Solvat davon nach einem der Ansprüche 1 bis 5 zur Verwendung in einem Verfahren zur Prophylaxe oder Behandlung von altersbedingter Makuladegeneration, Stargardt-Krankheit, Parodontitis, diabetischer Retinopathie, Glaukomen, Uveitis, rheumatoider Arthritis, Rückenmarksverletzungen, Schlaganfall, Multipler Sklerose, Parkinson-Krankheit, Alzheimer-Krankheit, Krebs und Atemwegserkrankungen, wie z.B. Asthma, chronisch obstruktiver Lungenerkrankung (COPD), allergischen Entzündungen, Emphysemen, Bronchitis, Bronchiektasie, Mukoviszidose, Tuberkulose, Lungenentzündung, Atemnotsyndrom (RDS - Neugeborene und Erwachsene), Rhinitis und Sinusitis; bakteriellen Infektionen, wie z.B. Sepsis, Ischämie-Reperfusionsschäden in verschiedenen Geweben, Myokardinfarkt, Anaphylaxie, paroxysmaler nächtlicher Hämoglobinurie, autoimmunen hämolytische Anämien, Psoriasis, Hidradenitis suppurativa, Myasthenia gravis, systemischem Lupus erythematodes, CHAPLE-Syndrom, C3-Glomeropathie, IgA-Nephropathie, atypischem hämolytisch-urämischem Syndrom, Morbus Crohn, Colitis ulcerosa oder dem Antiphospholipid-Syndrom.

## Revendications

1. Analogue de compstatine présentant la séquence : Ac-SEFA(1)|[1-Me-Trp]QDWGEHRhC(1)TEGE-[Peg3]ES-[K*]-NH₂
dans lequel les chaînes latérales de résidus désignés A(1) et hC(1) forment un pont de cystathionine, et
dans lequel K* est un résidu de lysine lié de manière covalente à un groupe Z¹- ou Z¹-Z²- ; dans lequel :
Z¹ est COOH-C₁₂₋₂₂alkylène-(CO)- ; et
Z² est choisi parmi :
[γGlu] ;
[γGlu][Peg3][Peg3] ;
[(Pipérazine-1-yl)acétyl][Peg3][Peg3] ;
[γGlu]G[γGlu] ;
[γGlu]K[γGlu] ;
[γGlu]KG[γGlu] ; ou
[Glu]G[Peg3][γGlu][Peg3]
où Peg3 représente l'acide 8-amino-3,6-dioxaoctanoïque ; ou
un sel et/ou un solvate pharmaceutiquement acceptable de celui-ci.

2. Analogue de compstatine ou sel et/ou solvate pharmaceutiquement acceptable de celui-ci selon la revendication 1, dans lequel Z¹ est HOOC- (CH₂)₁₂₋₂₂-(CO)-.

3. Analogue de compstatine ou sel et/ou solvate pharmaceutiquement acceptable de celui-ci selon la revendication 1 ou la revendication 2, dans lequel Z¹ est choisi parmi :
HOOC-(CH₂)₁₂-(CO)- ;
HOOC-(CH₂)₁₄-(CO)- ;
HOOC-(CH₂)₁₆-(CO)- ;
HOOC-(CH₂)₁₈-(CO)- ; ou
HOOC-(CH₂)₂₀-(CO)- ; et
dans lequel Z² est choisi parmi :
[γGlu] ;
[γGlu][Peg3][Peg3]- ;
[(Pipérazine-1-yl)acétyl][Peg3][Peg3] ;
[γGlu]G[γGlu] ;
[γGlu]K[γGlu] ;
[γGlu]KG[γGlu] ; ou
[γGlu]G[Peg3][γGlu][Peg3].

4. Analogue de compstatine ou sel et/ou solvate pharmaceutiquement acceptable de celui-ci selon l'une quelconque des revendications 1 à 3, dans lequel Z¹- ou Z¹-Z²- est choisi parmi :
15-carboxy-pentadécanoyle ;
15-carboxy-pentadécanoyle[γGlu],
15-carboxy-pentadécanoyle[γGlu][Peg3][Peg3] ;
19-carboxy-nonadécanoyle[γGlu][Peg3][Peg3] ;
15-carboxy-pentadécanoyl[(pipérazine-1-yl)acétyle][Peg3][Peg3] ;
17-carboxy-heptadécanoyle[γGlu]G[γGlu] ;
17-carboxy-heptadécanoyle[γGlu]K[γGlu] ;
17-carboxy-heptadécanoyle[γGlu]KG[γGlu] ;
17-carboxy-heptadécanoyle[γGlu]G[Peg3][γGlu][Peg3] ;
15-carboxy-pentadécanoyle[γGlu]G[γGlu] ;
17-carboxy-heptadécanoyle ;
17-carboxy-heptadécanoyle[γGlu] ;
19-carboxy-nonadécanoyle[γGlu]G[γGlu] ; et
17-carboxy-heptadécanoyle[γGlu][Peg3][Peg3].

5. Analogue de compstatine selon la revendication 1, qui est :
Ac-SEFA(1)|[1-Me-Trp]QDWGEHRhC(1)TEGE[Peg3]ES-K([17- carboxy-heptadécanoyle][γGlu])-NH²
dans lequel les chaînes latérales de résidus désignés A(1) et hC(1) forment un pont de cystathionine, ou un sel et/ou un solvate pharmaceutiquement acceptable de celui-ci.

6. Composition comprenant un analogue de compstatine selon l'une quelconque des revendications 1 à 5, ou un sel et/ou un solvate pharmaceutiquement acceptable de celui-ci, mélangé avec un support.

7. Composition selon la revendication 6, dans laquelle la composition est une composition pharmaceutique et le support est un support pharmaceutiquement acceptable.

8. Composition pharmaceutique comprenant un analogue de compstatine selon l'une quelconque des revendications 1 à 5, ou un sel et/ou un solvate pharmaceutiquement acceptable de celui-ci, mélangé avec un support, un excipient ou un véhicule pharmaceutiquement acceptable.

9. Analogue de compstatine, ou sel et/ou solvate pharmaceutiquement acceptable de celui-ci, selon l'une quelconque des revendications 1 à 5, pour utilisation en thérapie.

10. Analogue de compstatine, ou sel et/ou solvate pharmaceutiquement acceptable de celui-ci, selon l'une quelconque des revendications 1 à 5 pour utilisation dans un procédé de prophylaxie ou de traitement de la dégénérescence maculaire liée à l'âge, de la maladie de Stargardt, de la parodontite, de la rétinopathie diabétique, du glaucome, de l'uvéite, de la polyarthrite rhumatoïde, de la lésion de la moelle épinière, de l'accident vasculaire cérébral, de la sclérose en plaques, de la maladie de Parkinson, de la maladie d'Alzheimer, du cancer et des troubles respiratoires tels que l'asthme, la maladie pulmonaire obstructive chronique (MPOC), l'inflammation allergique, l'emphysème, la bronchite, la bronchectasie, la fibrose kystique, la tuberculose, la pneumonie, le syndrome de détresse respiratoire (RDS - néonatal et adulte), la rhinite et la sinusite ; des infections bactériennes telles que la septicémie, la lésion d'ischémie-reperfusion dans divers tissus, l'infarctus du myocarde, l'anaphylaxie, l'hémoglobinurie paroxysmique nocturne, l'anémie auto-immune hémolytique, le psoriasis, l'hidrosadénite suppurée, la myasthénie grave, le lupus érythémateux disséminé, le syndrome de CHAPLE, la glomérulopathie C3, la néphropathie à IgA, le syndrome hémolytique et urémique atypique,la maladie de Crohn, la colite ulcérative ou le syndrome des antiphospholipides.
